(19) Europäisches Patentamt
European Patent Office
Office européen des brevets

(11) Veröffentlichungsnummer: **0 206 225**
**A2**

(12) **EUROPÄISCHE PATENTANMELDUNG**

(21) Anmeldenummer: **86108250.1**

(22) Anmeldetag: **17.06.86**

(51) Int. Cl.⁴: **C 07 D 495/04**, C 07 D 491/048, C 07 D 401/06, C 07 D 401/12 // (C07D495/04, 333:00, 221:00)

(30) Priorität: **27.06.85 DE 3522959**

(43) Veröffentlichungstag der Anmeldung: **30.12.86 Patentblatt 86/52**

(84) Benannte Vertragsstaaten: **AT BE CH DE FR GB IT LI NL SE**

(71) Anmelder: **Merck Patent Gesellschaft mit beschränkter Haftung, Frankfurter Strasse 250, D-6100 Darmstadt (DE)**

(72) Erfinder: **Böttcher, Henning, Dr., Soderstrasse 95, D-6100 Darmstadt (DE)**
Erfinder: **Hausberg, Hans-Heinrich, Dr., Odenwaldstrasse 30, D-6105 Ober-Ramstadt (DE)**
Erfinder: ***Seyfried, Christoph, Dr., Mathildenstrasse 6,* D-6104 Seeheim-Jugenheim (DE)**
Erfinder: **Minck, Klaus-Otto, Dr., Büchestrasse 8, D-6105 Ober-Ramstadt (DE)**

(54) **Indolderivate.**

(57) Neue Indolderivate der allgemeinen Formel I

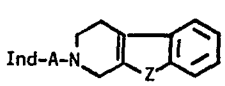

I

worin

Ind einen Indol-3-ylrest, der durch eine Hydroxymethyl-, Methylendioxy-, S-Alkyl-, SO-Alkyl-, $SO_2$-Alkyl-, CN- oder COW-Gruppe und/oder ein- oder zweifach durch Alkyl, O-Alkyl, OH, F, Cl oder Br substituiert sein kann,

W H, OH, O-Alkyl, $NH_2$, NH-Alkyl oder $N(Alkyl)_2$,

A $-(CH_2)_n-$, $-CH_2-S-CH_2CH_2-$, $-CH_2-SO-CH_2CH_2-$ oder $-CH_2-SO_2-CH_2CH_2-$,

Z $-(CH_2)_m-$, $-CH{=}CH-$, $-CHOH-$, $-CO-$, S, SO, $SO_2$ oder O,

n 2, 3, 4 oder 5 und

m 1, 2 oder 3

bedeuten,

worin die Alkylgruppen jeweils 1-4 C-Atome besitzen, sowie deren Salze zeigen Wirkungen auf das Zentralnervensystem.

EP 0 206 225 A2

Merck Patent Gesellschaft
mit beschränkter Haftung

6100 D a r m s t a d t

# Indolderivate

Die Erfindung betrifft neue Indolderivate der allgemeinen Formel I

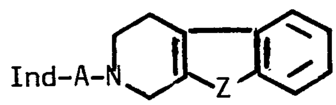

I

worin

Ind einen Indol-3-ylrest, der durch eine Hydroxymethyl-, Methylendioxy-, S-Alkyl-, SO-Alkyl-, $SO_2$-Alkyl-, CN- oder COW-Gruppe und/oder ein- oder zweifach durch Alkyl, O-Alkyl, OH, F, Cl oder Br substituiert sein kann,

W H, OH, OAlkyl, $NH_2$, NHAlkyl oder $N(Alkyl)_2$,

A $-(CH_2)_n-$, $-CH_2-S-CH_2CH_2-$, $-CH_2-SO-CH_2CH_2-$ oder $-CH_2-SO_2-CH_2CH_2-$,

Z $-(CH_2)_m-$, $-CH=CH-$, $-CHOH-$, $-CO-$, S, SO, $SO_2$ oder O,

n 2, 3, 4 oder 5,

m 1, 2 oder 3

bedeuten,

worin die Alkylgruppen jeweils 1 - 4 C-Atome besitzen,
sowie deren Salze.

Der Erfindung lag die Aufgabe zugrunde, neue Verbindungen aufzufinden, die zur Herstellung von Arzneimitteln verwendet werden können.

Es wurde gefunden, daß die Verbindungen der Formel I und ihre physiologisch unbedenklichen Salze wertvolle pharmakologische Eigenschaften besitzen. So zeigen sie insbesondere Wirkungen auf das Zentralnervensystem, vor allem dopaminstimulierende präsynaptische (neuroleptische) oder postsynaptische (Anti-Parkinson) Wirkungen. Im einzelnen induzieren die Verbindungen der Formel I contralaterales Drehverhalten in Hemiparkinson-Ratten (feststellbar nach der Methode von Ungerstedt et al., Brain Res. <u>24</u> (1970), 485-493) und hemmen die Bindung von tritiierten Dopaminagonisten und -antagonisten an striäre Rezeptoren (feststellbar nach der Methode von Schwarcz et al., J. Neurochemistry <u>34</u> (1980), 772-778, und Creese et al., European J. Pharmacol. <u>46</u> (1977), 377-381). Zusätzlich hemmen die Verbindungen den Zungen-Kieferreflex bei der narkotisierten Ratte (feststellbar in Anlehnung an die Methoden von Barnett et al., European J. Pharmacol. <u>21</u> (1973), 178-182, und von Ilhan et al., European J. Pharmacol. <u>33</u> (1975), 61-64). Weiterhin treten analgetische und blutdrucksenkende Wirkungen auf; so wird der bei katheter-tragenden wachen, spontan hypertonen Ratten (Stamm SHR/NIH-MO/CHB-EMD;

Methode vgl. Weeks und Jones, Proc. Soc. Exptl. Biol. Med. 104 (1960), 646-648) direkt gemessene Blutdruck nach intragastraler Gabe der Verbindungen gesenkt.

Verbindungen der Formel I und ihre physiologisch unbedenklichen Salze können daher als Arzneimittelwirkstoffe und auch als Zwischenprodukte zur Herstellung anderer Arzneimittelwirkstoffe verwendet werden.

Gegenstand der Erfindung sind die Indolderivate der Formel I sowie ihre Salze.

In den Resten Ind und W bedeutet Alkyl vorzugsweise Methyl, ferner auch Ethyl, n-Propyl, Isopropyl, n-Butyl, Isobutyl, sek.-Butyl oder tert.-Butyl. O-Alkyl ist vorzugsweise Methoxy, ferner auch Ethoxy, n-Propoxy, Isopropoxy, n-Butoxy, Isobutoxy, sek.-Butoxy oder tert.-Butoxy. S-Alkyl ist vorzugsweise Methylthio, ferner auch Ethylthio, Propylthio, Isopropylthio, Butylthio, Isobutylthio, sek.-Butylthio oder tert.-Butylthio. SO-Alkyl ist vorzugsweise Methylsulfinyl, ferner auch Ethyl-, Propyl-, Isopropyl-, Butyl-, Isobutyl-, sek.-Butyl- oder tert.-Butylsulfinyl, $SO_2$-Alkyl ist vorzugsweise Methylsulfonyl, ferner auch Ethyl-, Propyl-, Isopropyl-, Butyl-, Isobutyl-, sek.-Butyl- oder tert.-Butylsulfonyl.

Der Rest Ind bedeutet insbesondere einen unsubstituierten oder einen einfach substituierten Indol-3-ylrest. Vorzugsweise ist er in der 5- oder 6-Stellung oder auch in der 4- oder 7-Stellung substituiert. Weiterhin ist eine Substitution in 1- oder 2-Stellung möglich. Bevorzugte disubstituierte Indol-3-yl-reste sind in 5,6-Stellung substituiert; Disubstitution ist auch in 1,2-, 1,4-,

1,5-, 1,6-, 1,7-, 2,4-, 2,5-, 2,6-, 2,7-, 4,5-, 4,6-, 4,7-, 5,7- oder 6,7-Stellung möglich. In allen diesen Fällen können die Substituenten gleich oder verschieden sein.

Im einzelnen sind die bevorzugten Substituenten im Benzolring des Restes Ind Hydroxy und Methoxy, ferner Methyl, Ethyl, Ethoxy, F, Cl, Br, Methylthio, Methylsulfonyl, Methylsulfonyl, CN, Hydroxymethyl, Formyl, Carboxy, Methoxy- oder Ethoxycarbonyl, Carbamoyl, N-Methyl-, N-Ethyl-, N,N-Dimethyl- und N,N-Diethylcarbamoyl, weiterhin die übrigen oben angegebenen Alkyl-, O-Alkyl-, S-Alkyl-, SO-Alkyl oder $SO_2$-Alkylgruppen, ferner Propoxy-, Isopropoxy-, Butoxy-, Isobutoxy-, sek.-Butoxy- und tert.-Butoxycarbonyl, N-Propyl-, N-Isopropyl-, N-Butyl-, N-Isobutyl-, N-sek.-Butyl-, N-tert.-Butyl-, N-Methyl-N-ethyl-, N,N-Dipropyl-, N-Methyl-N-propyl-, N-Ethyl-N-propyl- und N,N-Dibutylcarbamoyl. Einige bevorzugte Bedeutungen des Restes Ind sind dementsprechend insbesondere 5-Hydroxyindol-3-yl, ferner 2-, 4-, 6- oder 7-Hydroxyindol-3-yl, das unsubstituierte Indol-3-yl, 2-, 4-, 5-, 6- oder 7-Methoxyindol-3-yl, weiterhin 1-, 2-, 4-, 5-, 6- oder 7-Methylindol-3-yl, 1-, 2-, 4-, 5-, 6- oder 7-Ethylindol-3-yl, 2-, 4-, 5-, 6- oder 7-Ethoxyindol-3-yl, 2-, 4-, 5-, 6- oder 7-Fluorindol-3-yl, 2-, 4-, 5-, 6- oder 7-Chlorindol-3-yl, 2-, 4-, 5-, 6- oder 7-Bromindol-3-yl, 2-, 4-, 5-, 6- oder 7-Methylthioindol-3-yl, 2-, 4-, 5-, 6- oder 7-Methylsulfinylindol-3-yl, 2-, 4-, 5-, 6- oder 7-Methylsulfonylindol-3-yl, 2-, 4-, 5-, 6- oder 7-Formyl-indol-3-yl, 2-, 4-, 5-, 6- oder 7-Carboxyindol-3-yl, 2-, 4-, 5-, 6- oder 7-Methoxycarbonylindol-3-yl, 2-, 4-, 5-, 6- oder 7-Ethoxycarbonylindol-3-yl, 2-, 4-, 5-, 6- oder 7-Carbamoylindol-3-yl, 2-, 4-, 5-, 6- oder 7-N-Methylcarbamoylindol-3-yl, 2-, 4-, 5-, 6- oder 7-N-Ethylcarbamoylindol-3-yl, 2-, 4-, 5-, 6- oder 7-N,N-Dimethylcarbamoylindol-3-yl, weiterhin 5,6-Dihydroxyindol-3-yl, 5-Hydroxy-6-methoxyindol-3-yl, 5-Methoxy-6-hydroxyindol-3-yl, 5,6-Dimethoxyindol-3-yl, ferner 4,5-, 4,6-, 4,7-, 5,7- oder 6,7-Dihydroxyindol-3-yl, 4-Hydroxy-5-, 6- oder 7-methoxyindol-3-yl, 5-Hydroxy-4- oder 7-methoxyindol-3-yl, 6-Hydroxy-4- oder 7-methoxyindol-3-yl, 7-Hydroxy-4-, 5- oder 6-methoxyindol-3-yl, 5,6-Methylendioxyindol-3-yl, 4,5-, 4,6- 4,7-, 5,7- oder 6,7-Dimethoxyindol-3-yl, 1-Methyl-4-, -5-, -6- oder -7-hydroxymethylindol-3-yl, 1-Methyl-4-, -5-, -6- oder -7-formylindol-3-yl, 1-Methyl-4-, -5-, -6- oder -7-carboxyindol-3-yl, 1-Methyl-4-, -5-, -6- oder -7-carbamoylindol-3-yl, 2-Methyl-4-, -5-, -6- oder -7-hydroxymethylindol-3-yl, 2-Methyl-4-, -5-, -6- oder -7-formylindol-3-yl, 2-Methyl-4-, -5-, -6- oder -7-carboxyindol-3-yl, 2-Methyl-4-, -5-, -6- oder -7-carbamoylindol-3-yl, 5-Methoxy-4-, -6- oder -7-methoxycarbonylindol-3-yl, 5-Methoxy-4-, -6- oder -7-ethoxycarbonylindol-3-yl, 5-Methoxy-4-, -6- oder -7-carboxyindol-3-yl, 5-Methoxy-4-, -6- oder -7-carbamoylindol-3-yl, 5-Fluor-4-, -6- oder -7-carboxyindol-3-yl, 5-Chlor-4-, -6- oder -7-carboxyindol-3-yl, 7-Chlor-4-, -5- oder -6-carboxyindol-3-yl, 5-Brom-4-, -6- oder -7-carboxyindol-3-yl, 5-Hydroxy-4-, -6- oder -7-methoxycarbonylindol-3-yl, 5-Hydroxy-4-, -6- oder -7-ethoxycarbonylindol-3-yl, 5-Hydroxy-4-, -6- oder -7-carboxyindol-3-yl, 5-Hydroxy-4-, -6- oder -7-carbamoylindol-3-yl, 5-Hydroxy-2-, -4-, -6- oder -7-hydroxymethylindol-3-yl, 6-Hydroxy-4-, -5- oder -7-carboxyindol-3-yl, 6-Hydroxy-2-, -4-, -5- oder -7-hydroxymethylindol-3-yl.

Der Parameter n ist vorzugsweise 4, der Rest A ist vorzugsweise $-(CH_2)_4-$ oder $-CH_2-S-CH_2CH_2-$, weiterhin vorzugsweise $-(CH_2)_2-$, $-(CH_2)_3-$ oder $-(CH_2)_5-$.

Der Parameter m ist vorzugsweise 2. Der Rest Z ist vorzugsweise $-(CH_2)_2-$, -CHOH-, S oder O.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis Il ausgedrückt werden, die der Formel I entsprechen und worin die nicht näher bezeichneten Reste und Paramter die bei Formel I angegebene Bedeutung haben, worin jedoch

| | | |
|---|---|---|
| in Ia | Ind | Hydroxyindol-3-yl, Methoxyindol-3-yl oder Indol-3-yl bedeutet, wobei die Substituenten vorzugsweise in 5- oder 6-Stellung stehen; |
| in Ib | Ind | 5- oder 6-Hydroxyindol-3-yl, 5- oder 6-Methoxyindol-3-yl oder Indol-3-yl bedeutet; |
| in Ic | A | $-(CH_2)_n-$ oder $-CH_2-S-CH_2CH_2-$ bedeutet; |
| in Id | A | $-(CH_2)_4-$ bedeutet; |
| in Ie | Z | $-(CH_2)_2-$ bedeutet; |
| in Ie' | Z | -CH=CH- bedeutet; |

| | | |
|---|---|---|
| in If | Z | -CHOH- bedeutet; |
| in Ig | Z | S bedeutet; |
| in Ih | Z | O bedeutet; |
| in Ii | Ind | 5- oder 6-Hydroxyindol-3-yl, 5- oder 6-Methoxyindol-3-yl oder Indol-3-yl, |
| | A | $-(CH_2)_n-$ oder $-CH_2-S-CH_2CH_2-$ und |
| | Z | $-(CH_2)_2-$ bedeuten; |
| in Ij | Ind | 5- oder 6-Hydroxyindol-3-yl, 5- oder 6-Methoxyindol-3-yl oder Indol-3-yl, |
| | A | $-(CH_2)_n-$ oder $-CH_2-S-CH_2CH_2-$ und |
| | Z | -CHOH- bedeuten; |
| in Ik | Ind | 5- oder 6-Hydroxyindol-3-yl, 5- oder 6-Methoxyindol-3-yl oder Indol-3-yl, |
| | A | $-(CH_2)_n-$ oder $-CH_2-S-CH_2CH_2-$ und |
| | Z | S bedeuten; |
| in Il | Ind | 5- oder 6-Hydroxyindol-3-yl, 5- oder 6-Methoxyindol-3-yl oder Indol-3-yl, |
| | A | $-(CH_2)_n-$ oder $-CH_2-S-CH_2CH_2-$ und |
| | Z | O bedeuten. |

Die Verbindungen der Formel I können ein oder mehrere asymmetrische Kohlenstoffatome besitzen. Sie können daher als Racemate, falls mehrere asymmetrische Kohlenstoffatome vorhanden sind, auch als Gemische mehrerer Racemate sowie in verschiedenen optisch-aktiven Formen vorliegen.

Gegenstand der Erfindung ist ferner das in Patentanspruch 5 beschriebene Verfahren zur Herstellung der Verbindungen der Formel I sowie ihrer Salze.

Die Herstellung der Verbindungen der Formel I erfolgt im übrigen nach an sich bekannten Methoden, wie sie in der Literatur (z. B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Indolderivaten der Formel II ist $X^1$ vorzugsweise X; dementsprechend sind in den Verbindungen der Formel III $X^2$ und $X^3$ vorzugsweise zusammen NH. Der Rest X ist vorzugsweise Cl oder Br; er kann jedoch auch J, OH oder eine reaktionsfähig funktionell abgewandelte OH-Gruppe bedeuten, insbesondere Alkylsulfonyloxy mit

1-6 (z. B. Methansulfonyloxy) oder Arylsulfonyloxy mit 6-10 C-Atomen (z. B. Benzolsulfonyloxy, p-Toluolsulfonyloxy, 1- oder 2-Naphthalin-sulfonyloxy).

Dementsprechend sind die Indolderivate der Formel I insbesondere durch Umsetzung von Verbindungen der Formel Ind-A-Cl oder Ind-A-Br mit Verbindungen der Formel III, worin $X^2$ und $X^3$ zusammen eine NH-Gruppe bedeuten (nachstehend als IIIa bezeichnet) erhältlich.

Die Verbindungen der Formeln II und III sind zum Teil bekannt; die nicht bekannten Verbindungen der Formeln II und III können leicht analog zu den bekannten Verbindungen hergestellt werden. Verbindungen der Formel II (A = $-CH_2-S-CH_2CH_2-$) können z. B. hergestellt werden aus Mannich-Basen der Formel IV und Thiolen der Formel $HS-CH_2CH_2-X^1$, z. B. $HS-CH_2CH_2OH$. Die Sulfoxide und Sulfone der Formel II (A = $-CH_2-SO-CH_2CH_2-$ oder $-CH_2-SO_2-CH_2CH_2-$) sind durch Oxydation der Thioether (II, A = $-CH_2-S-CH_2CH_2-$) zugänglich. Primäre Alkohole der Formel Ind-A-OH sind z. B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel Ind-A-Hal. Die entsprechenden Sulfonyloxyverbindungen sind erhältlich aus den Alkoholen Ind-A-OH durch Umsetzung mit den entsprechenden Sulfonsäurechloriden.

Die Jodverbindungen der Formel Ind-A-J sind z. B. durch Einwirkung von Kaliumjodid auf die zugehörigen p-Toluolsulfonsäureester erhältlich. Die Amine der Formel Ind-$A-NH_2$ sind z. B. aus den Halogeniden mit Phthalimidkalium oder durch Reduktion der entsprechenden Nitrile herstellbar.

Die Verbindungen IIIa sind teilweise bekannt [vgl. Can. J.Chem. 52. 2316-26 (1984); Arch.Pharm. 309. 279-88 (1976)] und z. B. erhältlich durch Umsetzung von Verbindungen der Formel VI

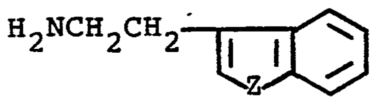

VI

mit Formaldehyd.

Verbindungen der Formel III ($X^2$ und $X^3$ = jeweils X) sind z. B. herstellbar durch Reduktion von Diestern der Formel VII

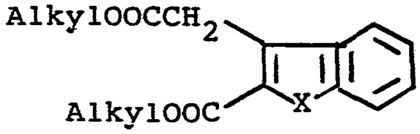

VII

zu Diolen der Formel III, $X^2 = X^3$ = OH und gegebenenfalls anschließende Umsetzung mit $SOCl_2$ bzw. $PBr_3$.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z. B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF)

oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente Ind-A-$NH_2$ bzw. IIIa kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z. B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben

angeführten Gruppen bzw. zusätzlichen Bindungen enthalten, reduktiv in eine Verbindung der Formel I überzuführen; dabei können gleichzeitig COW-Gruppen oder CO-Gruppen, die in der Ausgangsverbindung enthalten sind, reduziert werden. Vorzugsweise bedient man sich hierzu des nascierenden Wasserstoffs oder komplexer Metallhydride, ferner der Reduktion nach Wolff-Kishner.

Bevorzugte Ausgangsstoffe für die Reduktion entsprechen der Formel VIII

Ind'-L-Q VIII

worin

Ind' einen Indol-3-ylrest, der durch eine Hydroxymethyl-, Methylendioxy-, S-Alkyl-, SO-Alkyl-, $SO_2$-Alkyl-, CN- oder COW-Gruppe und/oder ein- oder zweifach durch Alkyl, O-Alkyl, OH, F, Cl, Br und/oder O-Benzyl und/oder durch eine Arylsulfonylgruppe oder eine Benzylgruppe in 1-Stellung substituiert sein kann,

L A oder eine dem Rest A entsprechende Kette, worin jedoch eine oder mehrere $-CH_2$-Gruppe(n) durch -CO- und/oder ein oder mehrere Wasserstoffatome durch OH-Gruppen ersetzt sind,

Q 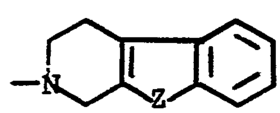 oder 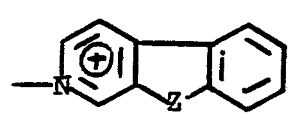 $An^{\ominus}$

$An^-$ ein Anion einer starken Säure bedeutet und

Z die oben angegebene Bedeutung hat,

worin jedoch nicht gleichzeitig Ind' = Ind, L = A und

Q = 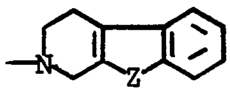 sein können.

In den Verbindungen der Formel VIII ist L bevorzugt $-CO-(CH_2)_{n-2}-CO-$ [im einzelnen $-COCO-$, $-COCH_2CO-$, $-CO-(CH_2)_2-CO-$, $-CO-(CH_2)_3-CO-$], $-(CH_2)_{n-1}-CO-$ [im einzelnen $-CH_2CO-$, $-CH_2CH_2-CO-$, $-(CH_2)_3-CO-$ oder $-(CH_2)_4-CO-$], $-CH_2-S-CH_2-CO-$, $-CH_2-SO-CH_2-CO-$ oder $-CH_2-SO_2-CH_2-CO-$, ferner z. B. $-CO-CH_2CH_2-$, $-CH_2-CO-CH_2-$, $-CO-(CH_2)_3-$, $-CH_2-CO-CH_2CH_2-$, $-CH_2CH_2-CO-CH_2-$, $-CO-(CH_2)_4-$, $-CH_2-CO-(CH_2)_3-$, $-CH_2CH_2-CO-CH_2CH_2-$ oder $-(CH_2)_3-CO-CH_2-$.

Verbindungen der Formel VIII sind z. B. herstellbar durch Umsetzung von IIIa oder einer Verbindung der Formel IX mit einer Verbindung der Formel X

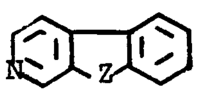 IX $Ind'-L-X^1$ X

worin

Z, Ind', L und $X^1$ die oben angegebenen Bedeutungen haben,

unter den Bedingungen, die oben für die Umsetzung von II mit III angegeben sind.

Wird als Reduktionsmittel nascierender Wasserstoff verwendet, so kann man diesen z. B. durch Behandlung von Metallen mit schwachen Säuren oder mit Basen erzeugen. So kann man z. B. ein Gemisch von Zink mit Alkalilauge oder von Eisen mit Essigsäure verwenden. Geeignet ist

auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalisch-wässeriger Lösung, gegebenenfalls unter Zusatz von Ethanol, verwenden. Auch Natrium- oder Aluminiumamalgam in wässerig-alkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie $LiAlH_4$, $NaBH_4$, Diisobutylaluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit $NaBH_4$ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden oder vinylogen Säureamiden (z. B. solchen der Formel VIII, worin L eine $-(CH_2)_{n-1}-CO-$, $-CH_2-S-CH_2-CO-$ oder $-CO-(CH_2)_{n-2}-CO-$Gruppe bedeutet) mit $LiAlH_4$ in THF bei Temperaturen zwischen etwa 0 und 66° zu $CH_2$-Gruppen reduzieren. Dabei können in 1-Stellung des Indolrings befindliche Arylsulfonyl-Schutzgruppen gleichzeitig reduktiv abgespalten und/oder Gruppen Z = CO und/oder am Indolring

befindliche COW-Gruppen reduziert werden, z. B. CO-Gruppen zu CHOH-Gruppen und/oder COOAlkyl-, COOH- oder CHO-Gruppen zu $CH_2OH$-Gruppen.

Eine Reduktion der Pyridiniumsalze der Formel VIII (worin Q 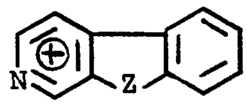 $An^{\ominus}$ und An vorzugsweise Cl, Br oder $CH_3SO_3$ bedeutet) zu Verbindungen der Formel I gelingt z. B. mit $NaBH_4$ in Wasser, Methanol oder Ethanol oder in Gemischen dieser Lösungsmittel, falls erwünscht unter Zusatz einer Base wie NaOH, bei Temperaturen zwischen etwa 0 und 80°.

N-Benzylgruppen können reduktiv mit Natrium in flüssigem Ammoniak abgespalten werden.

Es ist ferner möglich, eine oder mehrere Carbonylgruppen nach der Methode von Wolff-Kishner zu $CH_2$-Gruppen zu reduzieren, z. B. durch Behandlung mit wasserfreiem Hydrazin in absolutem Ethanol unter Druck bei Temperaturen zwischen etwa 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3-4 Stunden gekocht. Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Verbindungen, die sonst der Formel I entsprechen, aber an Stelle eines oder mehrerer H-Atome eine oder mehrere solvolysierbare Gruppe(n) enthalten, können zu den Verbindungen der Formel I solvolysiert, insbesondere hydrolysiert werden.

Die Ausgangsstoffe für die Solvolyse sind beispielsweise erhältlich durch Reaktion von IIIa mit Verbindungen, die der Formel II ($X^1$ = X) entsprechen, aber an Stelle eines oder mehrerer H-Atome eine oder mehrere solvolysierbare Gruppe(n) enthalten. So können insbesondere 1-Acylindolderivate (entsprechend der Formel I, aber in 1-Stellung des Ind-Rests eine Acylgruppe enthaltend, vorzugsweise eine Alkanoyl-, Alkylsulfonyl- oder Arylsulfonylgruppe mit jeweils bis zu 10 C-Atomen, wie Methan-, Benzol- oder p-Toluolsulfonyl) zu den entsprechenden in der 1-Stellung des Indolringes unsubstituierten Indolderivaten hydrolysiert werden, z. B. in saurem, besser in neutralem oder alkalischem Medium bei Temperaturen zwischen 0 und 200°. Analog gelingt eine Hydrolyse von Verbindungen, die der Formel I entsprechen, aber an Stelle der Gruppe Z eine CHCl-, CHBr- oder CHOAcyl-Gruppe (Acyl = z. B. Alkanoyl, Aroyl, Alkyl- oder Arylsulfonyl mit jeweils bis zu 10 C-Atomen) enthalten, zu Verbindungen der Formel I (Z = CHOH).

Als basische Katalysatoren verwendet man dabei zweckmäßig Natrium-, Kalium- oder Calciumhydroxid, Natrium- oder Kaliumcarbonat, oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze. Ein besonders schonendes Verfahren ist die Spaltung von Trialkylsilylethern, insbesondere

Trimethyl- oder Dimethyl-tert.-butyl-silylethern, mit Hilfe von KF in Methanol bei etwa 10 - 30°, wobei die entsprechenden Hydroxyverbindungen erhalten werden.

Indolderivate der Formel I (A = $-CH_2-S-CH_2CH_2-$) sind ferner durch Umsetzung von Mannich-Basen der Formel IV mit Thiolen der Formel V (oder ihren Salzen) erhältlich.

Die Ausgangsstoffe der Formeln IV und V sind teilweise bekannt; die nicht bekannten unter diesen Ausgangsstoffen können leicht analog zu den bekannten Verbindungen hergestellt werden. So sind die Mannich-Basen der Formel IV z. B. aus Indolen der Formel Ind-H, Formaldehyd und Aminen der Formel $HN(R\ )_2$ erhältlich, die Thiole der Formel V aus den Basen der Formel IIIa und Thiolderivaten der Formel $HS-CH_2CH_2-X^1$ (wobei die HS-Gruppe auch intermediär geschützt werden kann).

Im einzelnen erfolgt die Umsetzung von IV mit V in Gegenwart oder Abwesenheit eines inerten Lösungsmittels bei Temperaturen zwischen etwa -20 und 250°, vorzugsweise zwischen 60 und 150°. Als Lösungsmittel eignen sich beispielsweise Kohlenwasserstoffe wie Benzol, Toluol, Xylole oder Mesitylen; tertiäre Basen wie Triethylamin, Pyridin oder Picoline; Alkohole wie Methanol, Ethanol oder Butanol; Glykole und Glykolether wie Ethylenglykol, Diethylenglykol, 2-Methoxy-ethanol; Ketone wie Aceton; Ether wie THF oder Dioxan; Amide wie DMF; Sulfoxide wie Dimethylsulfoxid. Auch Gemische dieser Lösungsmittel sind geeignet. Die Thiole der Formel V werden zweckmäßig zunächst in die entsprechenden Mercaptide umgewandelt, vorzugsweise durch Reaktion mit Natrium- oder Kaliumhydroxid, Natrium- oder Kaliumethylat in die entsprechenden Natrium- oder Kaliummercaptide.

Weiterhin kann man gegebenenfalls eine Verbindung der Formel I nach an sich bekannten Methoden in eine andere Verbindung der Formel I umwandeln.

So kann man in einem Thioether der Formel I (A = $-CH_2-S-CH_2CH_2-$ oder Z = S) die Thioethergruppe zu einer SO-Gruppe oder zu einer $SO_2$-Gruppe oder in einem Sulfoxid der Formel I (A = $-CH_2-SO-CH_2CH_2-$ oder Z = SO) die SO-Gruppe zu einer $SO_2$-Gruppe oxydieren. Will man die Sulfoxide erhalten, so oxydiert man beispielsweise mit Wasserstoffperoxid, Persäuren wie m-Chlorperbenzoesäure, Cr(VI)-Verbindungen wie Chromsäure, $KMnO_4$, 1-Chlorbenztriazol, Ce(IV)-Verbindungen wie $(NH_4)_2Ce(NO_3)_6$, negativ substituierten aromatischen Diazoniumsalzen wie o- oder p-Nitrophenyldiazoniumchlorid oder elektrolytisch unter verhältnismäßig milden Bedingungen und bei relativ niedrigen Temperaturen (etwa -80 bis +100°). Will man dagegen die Sulfone (aus den Thioethern oder den Sulfoxiden) erhalten, so verwendet man die gleichen Oxydationsmittel unter kräftigeren Bedingungen und/oder im Überschuß sowie in der Regel bei höheren Temperaturen. Bei diesen Umsetzungen können die üblichen inerten Lösungsmittel zugegen oder abwesend sein. Als inerte Lösungsmittel eignen sich beispielsweise Wasser, wässerige Mineralsäuren, wässerige Alkalilaugen, niedere Alkohole wie Methanol oder Ethanol, Ester wie Ethylacetat, Ketone wie Aceton, niedere Carbonsäuren wie Essigsäure, Nitrile wie Acetonitril, Kohlenwasserstoffe wie Benzol, chlorierte Kohlenwasserstoffe wie Chloroform oder $CCl_4$. Ein bevorzugtes Oxydationsmittel ist 30 %iges wässeriges Wasserstoffperoxid. Dieses führt bei Anwendung der berechneten Menge in Lösungsmitteln wie Essigsäure, Aceton, Methanol, Ethanol oder wässeriger Natronlauge bei Temperaturen zwischen -20 und 100° zu den Sulfoxiden, im Überschuß bei höheren Temperaturen, vorzugsweise in Essigsäure oder in einem Gemisch aus Essigsäure und Acetanhydrid, zu den Sulfonen.

Ether der Formel I, in denen der Rest Ind ein- oder zweifach durch O-Alkyl substituiert ist, können gespalten werden, wobei die entsprechenden Hydroxyderivate entstehen. Z. B. kann man die Ether spalten durch Behandeln mit Dimethylsulfid-Bortribromid-Komplex, z. B. in Toluol, Ethern wie THF oder Dimethylsulfoxid, oder durch Verschmelzen mit Pyridin- oder Anilin-hydrohalogeniden, vorzugsweise Pyridinhydrochlorid, bei etwa 150-250°, oder durch Behandeln mit Diisobutylaluminiumhydrid in Toluol bei etwa 0-110°.

Weiterhin kann man nach an sich bekannten Methoden COW-Gruppen oder CN-Gruppen in (andere) COW-Gruppen umwandeln. So ist es möglich, Aldehydgruppen zu Carboxylgruppen zu oxydieren, beispielsweise mit $MnO_2$ in einem inerten Lösungsmittel wie Dichlormethan. Andererseits kann man Carboxylgruppen reduzieren, z. B. mit Diisobutylaluminiumhydrid in Toluol. Carboxylgruppen können verestert werden, z. B. durch Behandeln mit Alkoholen in Gegenwart eines sauren Katalysators oder durch Reaktion mit Diazoalkanen. Umwandlung der Carbonsäuren in ihre Chloride, z. B. mit $SOCl_2$, und nachfolgende Reaktion mit $NH_3$ oder Aminen führt zu den entsprechenden Carboxamiden, die auch durch Behandeln der Carbonsäureester mit Ammoniak oder Aminen erhältlich sind. Eine Solvolyse, vorzugsweise eine Hydrolyse unter den oben angegebenen Bedingungen, führt von Nitrilen zu Carbonsäureamiden oder von Nitrilen, Estern oder Amiden zu Carbonsäuren; insbesondere sind Carbonsäuren aus den Carbamoylverbindungen erhältlich, indem man diese mit NaOH oder KOH in wässerigen Glykolen oder Glykolethern, z. B. Diethylenglykolmonomethyl- oder -ethylether behandelt, zweckmäßig bei Temperaturen zwischen etwa 50 und etwa 200°.

Eine Reduktion von COW-, insbesondere Formyl-, Alkoxycarbonyl- oder Carboxylgruppen kann auch zu Hydroxymethylgruppen führen; analog können Gruppen Z = CO zu Gruppen Z = CHOH reduziert werden. Zweckmäßig verwendet man als Reduktionsmittel ein komplexes Hydrid wie $LiAlH_4$; Aldehyde und Ester können auch mit anderen der oben aufgeführten Reduktionsmittel reduziert werden. Vorzugsweise arbeitet man unter den oben angegebenen Bedingungen. Umgekehrt kann man Hydroxymethylgruppen bzw. CHOH-Gruppen, z. B. mit $MnO_2$ oder $Ba(MnO_4)_2$, zu Formyl- oder Carboxylgruppen bzw. zu CO-Gruppen oxydieren.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z. B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z. B. Pikrate, können zur Isolierung oder Aufreinigung der Verbindungen der Formel I verwendet werden.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Andererseits können Verbindungen der Formel I mit freien COOH-Gruppen durch Umsetzung mit Basen in ihre Metall- bzw. Ammoniumsalze umgewandelt werden. Als Salze kommen insbesondere die Natrium-, Kalium-, Magnesium-, Calcium- und Ammoniumsalze in Betracht, ferner substituierte Ammoniumsalze, z. B. die Dimethyl-, Diethyl-, Monoethanol-, Diethanol-, Triethanol-, Cyclohexyl- oder Dicyclohexylammoniumsalze.

Die Verbindungen der Formel I und ihre physiologisch unbedenklichen Salze können zur Herstellung pharmazeutischer Zubereitungen verwendet werden, insbesondere auf nichtchemischem Wege. Hierbei können sie zusammen mit mindestens einem festen, flüssigen oder halb-flüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate

wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre physiologisch unbedenklichen Salze können an Menschen oder Tiere, insbesondere Säugetiere wie Affen, Hunde , Katzen, Ratten oder Mäuse verabreicht und bei der therapeutischen Behandlung des menschlichen oder tierischen Körpers und bei der Bekämpfung von Krankheiten verwendet werden, insbesondere bei der Therapie von Parkinsonismus, von extrapyramidalen Störungen bei der Neuroleptikatherapie, von Depressionen und/oder Psychosen und von Nebenwirkungen bei der Behandlung der Hypertonie (z. B. mit $\alpha$-Methyldopa). Ferner können die Verbindungen in der Endokrinologie und Gynäkologie Verwendung finden, z. B. zur Therapie von Akromegalie, Hypogonadismus, sekundärer Amenorrhoe, prämenstruellem Syndrom, unerwünschter puerperaler Laktation und generell als Prolaktin-Hemmer, weiterhin zur Therapie cerebraler Störungen (z. B. Migräne), insbesondere in der Geriatrie ähnlich wie gewisse Ergot-Alkaloide, sowie auch zur Blutdrucksenkung.

Dabei werden die erfindungsgemäßen Substanzen in der Regel in Analogie zu bekannten, im Handel befindlichen Präparaten (z. B. Bromocriptin, Dihydroergocornin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkte und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben. Rf-Werte wurden dünnschichtchromatographisch an Kieselgel ermittelt.

Beispiel 1

Man rührt eine Lösung von 20,8 g 3-(4-Chlorbutyl)-indol [oder 25,2 g 3-(4-Brombutyl)-indol] und 18,9 g 1,2,3,4-Tetrahydrobenzothieno[2,3-c]pyridin in 100 ml Acetonitril 12 Stunden bei 20°, arbeitet wie üblich auf und erhält 2-[4-(Indol-3-yl)-butyl]-1,2,3,4-tetrahydro-benzothieno[2,3-c]pyridin ("A"), F. 113 - 115°.

Analog erhält man mit den entsprechenden Indolderivaten der Formel II ($X^1$ = X) die folgenden 1,2,3,4-Tetrahydrobenzothieno[2,3-c]pyridine:

2-[2-(Indol-3-yl)-ethyl]-
2-[3-(Indol-3-yl)-propyl]-
2-[4-(4-Cyanindol-3-yl)-butyl]-
2-[4-(5-Cyanindol-3-yl)-butyl]-
2-[4-(6-Cyanindol-3-yl)-butyl]-
2-[4-(7-Cyanindol-3-yl)-butyl]-
2-[4-(4-Methoxycarbonylindol-3-yl)-butyl]-
2-[4-(5-Methoxycarbonylindol-3-yl)-butyl]-
2-[4-(6-Methoxycarbonylindol-3-yl)-butyl]-
2-[4-(7-Methoxycarbonylindol-3-yl)-butyl]-
2-[4-(4-Ethoxycarbonylindol-3-yl)-butyl]-
2-[4-(5-Ethoxycarbonylindol-3-yl)-butyl]-
2-[4-(6-Ethoxycarbonylindol-3-yl)-butyl]-
2-[4-(7-Ethoxycarbonylindol-3-yl)-butyl]-
2-[4-(4-Carbamoylindol-3-yl)-butyl]-
2-[4-(5-Carbamoylindol-3-yl)-butyl]-
2-[4-(6-Carbamoylindol-3-yl)-butyl]-
2-[4-(7-Carbamoylindol-3-yl)-butyl]-
2-[4-(5-N-Methylcarbamoylindol-3-yl)-butyl]-
2-[4-(5-N,N-Dimethylcarbamoylindol-3-yl)-butyl]-2-[4-(1-Methylindol-3-yl)-butyl]-
2-[4-(2-Methylindol-3-yl)-butyl]-
2-[4-(5-Methylindol-3-yl)-butyl]-
2-[4-(4-Methoxyindol-3-yl)-butyl]-
2-[4-(5-Methoxyindol-3-yl)-butyl]-, Rf 0,45 (Ethylacetat)
2-[4-(6-Methoxyindol-3-yl)-butyl]-
2-[4-(7-Methoxyindol-3-yl)-butyl]-
2-[4-(5,6-Dimethoxyindol-3-yl)-butyl]-
2-[4-(4-Hydroxyindol-3-yl)-butyl]-
2-[4-(5-Hydroxyindol-3-yl)-butyl]-, F. 181 - 183°
2-[4-(6-Hydroxyindol-3-yl)-butyl]-
2-[4-(7-Hydroxyindol-3-yl)-butyl]-
2-[4-(5,6-Dihydroxyindol-3-yl)-butyl]-
2-[4-(5-Fluorindol-3-yl)-butyl]-
2-[4-(6-Fluorindol-3-yl)-butyl]-
2-[4-(5-Chlorindol-3-yl)-butyl]-
2-[4-(6-Chlorindol-3-yl)-butyl]-
2-[4-(5-Bromindol-3-yl)-butyl]-
2-[4-(6-Bromindol-3-yl)-butyl]-
2-[5-(Indol-3-yl)-pentyl]-.

Beispiel 2

Analog Beispiel 1 erhält man mit 1,2,3,4-Tetrahydrobenzofuro[2,3-c]-pyridin [F. 109° (Zers.); erhältlich durch Reaktion von 1-Benzyl-3-piperidon mit O-Phenylhydroxylamin zu 2-Benzyl-1,2,3,4-tetrahydrobenzofuro-[2,3-c]pyridin und Hydrogenolyse] 2-[4-(Indol-3-yl)-butyl]-1,2,3,4-tetrahydrobenzofuro[2,3-c]pyridin.

Analog erhält man die folgenden 1,2,3,4-Tetrahydrobenzofuro[2,3-c]-pyridine:

3-[2-(Indol-3-yl)-ethyl]-
3-[3-(Indol-3-yl)-propyl]-3-[4-(4-Cyanindol-3-yl)-butyl]-
3-[4-(5-Cyanindol-3-yl)-butyl]-
3-[4-(6-Cyanindol-3-yl)-butyl]-
3-[4-(7-Cyanindol-3-yl)-butyl]-
3-[4-(4-Methoxycarbonylindol-3-yl)-butyl]-
3-[4-(5-Methoxycarbonylindol-3-yl)-butyl]-
3-[4-(6-Methoxycarbonylindol-3-yl)-butyl]-
3-[4-(7-Methoxycarbonylindol-3-yl)-butyl]-
3-[4-(4-Ethoxycarbonylindol-3-yl)-butyl]-
3-[4-(5-Ethoxycarbonylindol-3-yl)-butyl]-
3-[4-(6-Ethoxycarbonylindol-3-yl)-butyl]-
3-[4-(7-Ethoxycarbonylindol-3-yl)-butyl]-
3-[4-(4-Carbamoylindol-3-yl)-butyl]-
3-[4-(5-Carbamoylindol-3-yl)-butyl]-
3-[4-(6-Carbamoylindol-3-yl)-butyl]-
3-[4-(7-Carbamoylindol-3-yl)-butyl]-
3-[4-(5-N-Methylcarbamoylindol-3-yl)-butyl]-
3-[4-(5-N,N-Dimethylcarbamoylindol-3-yl)-butyl]-
3-[4-(1-Methylindol-3-yl)-butyl]-
3-[4-(2-Methylindol-3-yl)-butyl]-
3-[4-(5-Methylindol-3-yl)-butyl]-
3-[4-(4-Methoxyindol-3-yl)-butyl]-
3-[4-(5-Methoxyindol-3-yl)-butyl]-, Rf 0,5 (Ethylacetat)
3-[4-(6-Methoxyindol-3-yl)-butyl]-
3-[4-(7-Methoxyindol-3-yl)-butyl]-
3-[4-(5,6-Dimethoxyindol-3-yl)-butyl]-
3-[4-(4-Hydroxyindol-3-yl)-butyl]-
3-[4-(5-Hydroxyindol-3-yl)-butyl]-, F. 173 - 175°
3-[4-(6-Hydroxyindol-3-yl)-butyl]-
3-[4-(7-Hydroxyindol-3-yl)-butyl]-
3-[4-(5,6-Dihydroxyindol-3-yl)-butyl]-
3-[4-(5-Fluorindol-3-yl)-butyl]-
3-[4-(6-Fluorindol-3-yl)-butyl]-
3-[4-(5-Chlorindol-3-yl)-butyl]-3-[4-(6-Chlorindol-3-yl)-butyl]-
3-[4-(5-Bromindol-3-yl)-butyl]-
3-[4-(6-Bromindol-3-yl)-butyl]-
3-[5-(Indol-3-yl)-pentyl].

Beispiel 3

Analog Beispiel 1 erhält man mit 1,2,3,4,5,6-Hexahydro-benz[f]isochinolin das 3-[4-(Indol-3-yl)-butyl]-1,2,3,4,5,6-hexahydro-benz[f]isochinolin, F. 190-192 °. Hydrochlorid, F. 198-200 °.

Analog erhält man die folgenden 1,2,3,4,5,6-Hexahydro-benz[f]isochinoline:

3-[2-(Indol-3-yl)-ethyl]-
3-[3-(Indol-3-yl)-propyl]-
3-[4-(5,6-Methylendioxyindol-3-yl)-butyl]-, F. 133-135 °
3-[4-(4-Cyanindol-3-yl)-butyl]-
3-[4-(5-Cyanindol-3-yl)-butyl]-
3-[4-(6-Cyanindol-3-yl)-butyl]-
3-[4-(7-Cyanindol-3-yl)-butyl]-
3-[4-(4-Methoxycarbonylindol-3-yl)-butyl]-
3-[4-(5-Methoxycarbonylindol-3-yl)-butyl]-
3-[4-(6-Methoxycarbonylindol-3-yl)-butyl]-
3-[4-(7-Methoxycarbonylindol-3-yl)-butyl]-
3-[4-(4-Ethoxycarbonylindol-3-yl)-butyl]-
3-[4-(5-Ethoxycarbonylindol-3-yl)-butyl]-
3-[4-(6-Ethoxycarbonylindol-3-yl)-butyl]-
3-[4-(7-Ethoxycarbonylindol-3-yl)-butyl]-
3-[4-(4-Carbamoylindol-3-yl)-butyl]-
3-[4-(5-Carbamoylindol-3-yl)-butyl]-
3-[4-(6-Carbamoylindol-3-yl)-butyl]-
3-[4-(7-Carbamoylindol-3-yl)-butyl]-3-[4-(5-N-Methylcarbamoylindol-3-yl)-butyl]-
3-[4-(5-N,N-Dimethylcarbamoylindol-3-yl)-butyl]-
3-[4-(1-Methylindol-3-yl)-butyl]-
3-[4-(2-Methylindol-3-yl)-butyl]-
3-[4-(5-Methylindol-3-yl)-butyl]-
3-[4-(4-Methoxyindol-3-yl)-butyl]-
3-[4-(5-Methoxyindol-3-yl)-butyl]-
3-[4-(6-Methoxyindol-3-yl)-butyl]-
3-[4-(7-Methoxyindol-3-yl)-butyl]-
3-[4-(5,6-Dimethoxyindol-3-yl)-butyl]-
3-[4-(4-Hydroxyindol-3-yl)-butyl]-
3-[4-(5-Hydroxyindol-3-yl)-butyl]-, F. 184 - 186°
3-[4-(6-Hydroxyindol-3-yl)-butyl]-
3-[4-(7-Hydroxyindol-3-yl)-butyl]-
3-[4-(5,6-Dihydroxyindol-3-yl)-butyl]-
3-[4-(5-Fluorindol-3-yl)-butyl]-
3-[4-(6-Fluorindol-3-yl)-butyl]-
3-[4-(5-Chlorindol-3-yl)-butyl]-
3-[4-(6-Chlorindol-3-yl)-butyl]-
3-[4-(5-Bromindol-3-yl)-butyl]-
3-[4-(6-Bromindol-3-yl)-butyl]-
3-[5-(Indol-3-yl)-pentyl]-.

Beispiel 4

Analog Beispiel 1 erhält man mit 1,2,3,4-Tetrahydro-2-azafluoren [erhältlich durch Reaktion von 3-Methylinden mit Ammoniumacetat und Formaldehyd zu 4a-Methyl-1,2,3,4,4a,9a-hexahydro-2-aza-4-oxafluoren und Behandeln mit HCl] das 2-[4-(Indol-3-yl)-butyl]-1,2,3,4-tetrahydro-2-azafluoren.

Analog erhält man die folgenden 1,2,3,4-Tetrahydro-2-azafluorene:

2-[2-(Indol-3-yl)-ethyl]-
2-[3-(Indol-3-yl)-propyl]-
2-[4-(4-Cyanindol-3-yl)-butyl]-
2-[4-(5-Cyanindol-3-yl)-butyl]-
2-[4-(6-Cyanindol-3-yl)-butyl]-
2-[4-(7-Cyanindol-3-yl)-butyl]-
2-[4-(4-Methoxycarbonylindol-3-yl)-butyl]-
2-[4-(5-Methoxycarbonylindol-3-yl)-butyl]-
2-[4-(6-Methoxycarbonylindol-3-yl)-butyl]-
2-[4-(7-Methoxycarbonylindol-3-yl)-butyl]-
2-[4-(4-Ethoxycarbonylindol-3-yl)-butyl]-
2-[4-(5-Ethoxycarbonylindol-3-yl)-butyl]-
2-[4-(6-Ethoxycarbonylindol-3-yl)-butyl]-
2-[4-(7-Ethoxycarbonylindol-3-yl)-butyl]-
2-[4-(4-Carbamoylindol-3-yl)-butyl]-
2-[4-(5-Carbamoylindol-3-yl)-butyl]-
2-[4-(6-Carbamoylindol-3-yl)-butyl]-
2-[4-(7-Carbamoylindol-3-yl)-butyl]-
2-[4-(5-N-Methylcarbamoylindol-3-yl)-butyl]-
2-[4-(5-N,N-Dimethylcarbamoylindol-3-yl)-butyl]-
2-[4-(1-Methylindol-3-yl)-butyl]-
2-[4-(2-Methylindol-3-yl)-butyl]-
2-[4-(5-Methylindol-3-yl)-butyl]-
2-[4-(4-Methoxylindol-3-yl)-butyl]-
2-[4-(5-Methoxylindol-3-yl)-butyl]-, F. 143-145 °
2-[4-(6-Methoxylindol-3-yl)-butyl]-
2-[4-(7-Methoxylindol-3-yl)-butyl]-
2-[4-(5,6-Dimethoxyindol-3-yl)-butyl]-
2-[4-(4-Hydroxyindol-3-yl)-butyl]-, F. 198-201 °
2-[4-(5-Hydroxyindol-3-yl)-butyl]-
2-[4-(6-Hydroxyindol-3-yl)-butyl]-
2-[4-(7-Hydroxyindol-3-yl)-butyl]-

2-[4-(5,6-Dihydroxyindol-3-yl)-butyl]-
2-[4-(5-Fluorindol-3-yl)-butyl]-
2-[4-(6-Fluorindol-3-yl)-butyl]-
2-[4-(5-Chlorindol-3-yl)-butyl]-
2-[4-(6-Chlorindol-3-yl)-butyl]-
2-[4-(5-Bromindol-3-yl)-butyl]-
2-[4-(6-Bromindol-3-yl)-butyl]-
2-[5-(Indol-3-yl)-pentyl]-.

Beispiel 5

Analog Beispiel 1 erhält man mit 1,2,3,4,6,7-Hexahydro-3-aza-5H-dibenzo[a,c]cyclohepten (erhältlich durch Reaktion von 3-Methyl-6,7-dihydro-5H-benzocyclohepten mit Ammoniumacetat und Formaldehyd zu 11b-Methyl-1,2,3,4,4a,6,7,11b-oktahydro-3-aza-1-oxa-5H-dibenzo[a,c]cyclohepten und Behandeln mit HCl) das 3-[4-(Indol-3-yl)-butyl]-1,2,3,4,6,7-hexahydro-3-aza-5H-dibenzo[a,c]-cyclohepten sowie das 3-[4-(5-Hydroxyindol-3-yl)-butyl]-1,2,3,4,6,7-hexahydro-3-aza-5H-dibenzo[a,c]cyclohepten, F. 119-121 °.

Beispiel 6

Analog Beispiel 1 erhält man mit 1,2,3,4-Tetrahydro-benzothieno[2,3-c]pyridin-5-oxid (erhältlich durch Kochen von 1,2,3,4-Tetrahydro-benzothieno[2,3-c]pyridin mit 30 %ig. $H_2O_2$ in Ethanol) das 2-[4-(Indol-3-yl)-butyl]-1,2,3,4-tetrahydro-benzothieno[2,3-c]pyridin-5-oxid.

Analog erhält man mit den entsprechenden Indolderivaten die S-Oxide der in Beispiel 1 genannten Verbindungen.

Beispiel 7

Analog Beispiel 1 erhält man mit 1,2,3,4-Tetrahydro-benzothieno[2,3-c]pyridin-S,S-dioxid (erhältlich durch Kochen von 1,2,3,4-Tetrahydro-benzothieno[2,3-c]pyridin mit 30 %ig. $H_2O_2$ in Essigsäure) das 2-[4-(Indol-3-yl)-butyl]-1,2,3,4-tetrahydro-benzothieno[2,3-c]pyridin-S,S-dioxid.

Analog erhält man mit den entsprechenden Indolderivaten die S,S-Dioxide der in Beispiel 1 genannten Verbindungen.

Beispiel 8

Analog Beispiel 1 erhält man mit 9-Hydroxy-1,2,3,4-tetrahydro-2-azafluoren (erhältlich durch Bromierung von 1,2,3,4-Tetrahydro-2-azafluoren mit Pyrrolidinhydrobromidperbromid zu 9-Brom-1,2,3,4-tetrahydro-2-azafluoren und Hydrolyse mit KOH) das 2-[4-(Indol-3-yl)-butyl]-9-hydroxy-1,2,3,4-tetrahydro-2-azafluoren, F. 205 - 208°.

Analog erhält man folgende 9-Hydroxy-1,2,3,4-tetrahydro-2-azafluorene:

2-[2-(Indol-3-yl)-ethyl]-
2-[3-(Indol-3-yl)-propyl]-
2-[4-(4-Cyanindol-3-yl)-butyl]-
2-[4-(5-Cyanindol-3-yl)-butyl]-
2-[4-(6-Cyanindol-3-yl)-butyl]-
2-[4-(7-Cyanindol-3-yl)-butyl]-
2-[4-(4-Methoxycarbonylindol-3-yl)-butyl]-
2-[4-(5-Methoxycarbonylindol-3-yl)-butyl]-
2-[4-(6-Methoxycarbonylindol-3-yl)-butyl]-
2-[4-(7-Methoxycarbonylindol-3-yl)-butyl]-2-[4-(4-Ethoxycarbonylindol-3-yl)-butyl]-
2-[4-(5-Ethoxycarbonylindol-3-yl)-butyl]-
2-[4-(6-Ethoxycarbonylindol-3-yl)-butyl]-
2-[4-(7-Ethoxycarbonylindol-3-yl)-butyl]-
2-[4-(4-Carbamoylindol-3-yl)-butyl]-
2-[4-(5-Carbamoylindol-3-yl)-butyl]-
2-[4-(6-Carbamoylindol-3-yl)-butyl]-
2-[4-(7-Carbamoylindol-3-yl)-butyl]-
2-[4-(5-N-Methylcarbamoylindol-3-yl)-butyl]-
2-[4-(5-N,N-Dimethylcarbamoylindol-3-yl)-butyl]-
2-[4-(1-Methylindol-3-yl)-butyl]-
2-[4-(2-Methylindol-3-yl)-butyl]-
2-[4-(5-Methylindol-3-yl)-butyl]-
2-[4-(4-Methoxyindol-3-yl)-butyl]-
2-[4-(5-Methoxyindol-3-yl)-butyl]-
2-[4-(6-Methoxyindol-3-yl)-butyl]-
2-[4-(7-Methoxyindol-3-yl)-butyl]-
2-[4-(5,6-Dimethoxyindol-3-yl)-butyl]-
2-[4-(4-Hydroxyindol-3-yl)-butyl]-
2-[4-(5-Hydroxyindol-3-yl)-butyl]-
2-[4-(6-Hydroxyindol-3-yl)-butyl]-
2-[4-(7-Hydroxyindol-3-yl)-butyl]-
2-[4-(5,6-Dihydroxyindol-3-yl)-butyl]-
2-[4-(5-Fluorindol-3-yl)-butyl]-
2-[4-(6-Fluorindol-3-yl)-butyl]-
2-[4-(5-Chlorindol-3-yl)-butyl]-
2-[4-(6-Chlorindol-3-yl)-butyl]-
2-[4-(5-Bromindol-3-yl)-butyl]-
2-[4-(6-Bromindol-3-yl)-butyl]-
2-[5-(Indol-3-yl)-pentyl]-.

Beispiel 9

Analog Beispiel 1 erhält man mit 9-Oxo-1,2,3,4-tetrahydro-2-azafluoren (erhältlich aus 9-Hydroxy-1,2,3,4-tetrahydro-2-azafluoren und $CrO_3$) das 2-[4-(Indol-3-yl)-butyl]-9-oxo-1,2,3,4-tetrahydro-2-azafluoren.

Analog erhält man folgende 9-Oxo-1,2,3,4-tetrahydro-2-azafluorene:

2-[2-(Indol-3-yl)-ethyl]-
2-[3-(Indol-3-yl)-propyl]-
2-[4-(4-Cyanindol-3-yl)-butyl]-
2-[4-(5-Cyanindol-3-yl)-butyl]-
2-[4-(6-Cyanindol-3-yl)-butyl]-
2-[4-(7-Cyanindol-3-yl)-butyl]-
2-[4-(4-Methoxycarbonylindol-3-yl)-butyl]-
2-[4-(5-Methoxycarbonylindol-3-yl)-butyl]-
2-[4-(6-Methoxycarbonylindol-3-yl)-butyl]-
2-[4-(7-Methoxycarbonylindol-3-yl)-butyl]-
2-[4-(4-Ethoxycarbonylindol-3-yl)-butyl]-
2-[4-(5-Ethoxycarbonylindol-3-yl)-butyl]-
2-[4-(6-Ethoxycarbonylindol-3-yl)-butyl]-
2-[4-(7-Ethoxycarbonylindol-3-yl)-butyl]-
2-[4-(4-Carbamoylindol-3-yl)-butyl]-
2-[4-(5-Carbamoylindol-3-yl)-butyl]-
2-[4-(6-Carbamoylindol-3-yl)-butyl]-
2-[4-(7-Carbamoylindol-3-yl)-butyl]-
2-[4-(5-N-Methylcarbamoylindol-3-yl)-butyl]-
2-[4-(5-N,N-Dimethylcarbamoylindol-3-yl)-butyl]-
2-[4-(1-Methylindol-3-yl)-butyl]-
2-[4-(2-Methylindol-3-yl)-butyl]-
2-[4-(5-Methylindol-3-yl)-butyl]-2-[4-(4-Methoxyindol-3-yl)-butyl]-
2-[4-(5-Methoxyindol-3-yl)-butyl]-
2-[4-(6-Methoxyindol-3-yl)-butyl]-
2-[4-(7-Methoxyindol-3-yl)-butyl]-
2-[4-(5,6-Dimethoxyindol-3-yl)-butyl]-
2-[4-(4-Hydroxyindol-3-yl)-butyl]-
2-[4-(5-Hydroxyindol-3-yl)-butyl]-
2-[4-(6-Hydroxyindol-3-yl)-butyl]-
2-[4-(7-Hydroxyindol-3-yl)-butyl]-
2-[4-(5,6-Dihydroxyindol-3-yl)-butyl]-
2-[4-(5-Fluorindol-3-yl)-butyl]-
2-[4-(6-Fluorindol-3-yl)-butyl]-
2-[4-(5-Chlorindol-3-yl)-butyl]-
2-[4-(6-Chlorindol-3-yl)-butyl]-
2-[4-(5-Bromindol-3-yl)-butyl]-
2-[4-(6-Bromindol-3-yl)-butyl]-
2-[5-(Indol-3-yl)-pentyl]-.

Beispiel 10

Ein Gemisch von 1,88 g 3-(4-Amino-butyl)-indol und 2,45 g 2-Chlormethyl-3-(2-chlorethyl)-benzothiophen (erhältlich durch Reduktion von Benzothiophen-2-carbonsäure-3-essigsäure-diethylester mit $LiAlH_4$ und anschließende Reaktion mit $SOCl_2$) in 40 ml Aceton und 40 ml Wasser wird 24 Stunden gekocht und wie üblich aufgearbeitet. Man erhält "A", F. 113 - 115°.

Beispiel 11

Zu einer Suspension von 23,4 g $LiAlH_4$ in 1100 ml absolutem THF tropft man unter Rühren eine Suspension von 40,2 g 2-[4-(5-Methoxyindol-3-yl)-1,4-dioxobutyl]-1,2,3,4-tetrahydro-benzofuro[2,3-c]pyridin [erhältlich aus

4-(5-Methoxy-indol-3-yl)-4-oxobuttersäure und 1,2,3,4-Tetrahydro-benzofuro[2,3-c]pyridin] in 3 l heißem absolutem THF, kocht 1 Std., kühlt ab, zersetzt mit Wasser und Natronlauge und arbeitet wie üblich auf. Man erhält 2-[4-(5-Methoxyindol-3-yl)-butyl]-1,2,3,4-tetrahydro-benzofuro[2,3-c]pyridin, Rf 0,5 (Ethylacetat).

Analog erhält man aus 2-[4-(2-Carboxyindol-3-yl)-1-oxo-butyl]-1,2,3,4-tetrahydro-benzofuro[2,3-c]pyridin:

2-[4-(2-Carboxyindol-3-yl)-butyl]-1,2,3,4-tetrahydro-benzofuro[2,3-c]pyridin.

Beispiel 12

Zu einer Lösung von 4,51 g 1-[4-(5-Methoxyindol-3-yl)-butyl]-benzofuro[2,3-c]pyridinium-bromid [erhältlich aus 3-(4-Brombutyl)-5-methoxyindol und Benzofuro[2,3-c]pyridin] in 50 ml 1n NaOH gibt man unter Rühren 1 g $NaBH_4$ in 20 ml Wasser und rührt danach noch 3 Stunden bei 60°. Nach üblicher Aufarbeitung erhält man 2-[4-(5-Methoxyindol-3-yl)-butyl]-1,2,3,4-tetrahydro-benzofuro-[2,3-c]pyridin, Rf 0,5 (Ethylacetat).

Beispiel 13

Analog Beispiel 12 erhält man aus 2-[4-(Indol-3-yl)-butyl]-9-oxo-indeno[2,3-c]pyridiniumbromid [erhältlich aus 3-(4-Brombutyl)-indol und 2-Azafluorenon] und überschüssigem $NaBH_4$ das 2-[4-(Indol-3-yl)-butyl]-9-hydroxy-1,2,3,4-tetrahydro-2-azafluoren, F. 205 - 208°.

Beispiel 14

Ein Gemisch von 36,9 g 2-[4-(5-Cyanindol-3-yl)-butyl]-1,2,3,4-tetrahydro-benzofuro[2,3-c]pyridin (erhältlich aus der entsprechenden 5-Formylverbindung über das Oxim), 27,1 g NaOH, 520 ml Wasser und 420 ml Diethylenglykolmonoethylether wird 3 Std. bei 140° Badtemperatur gerührt. Man kühlt ab, arbeitet wie üblich auf und erhält 2-[4-(5-Carbamoylindol-3-yl)-butyl]-1,2,3,4-tetrahydro-benzofuro[2,3-c]pyridin.

Beispiel 15

Man arbeitet wie in Beispiel 14 beschrieben, kocht aber 16 Std. und erhält nach üblicher Aufarbeitung 2-[4-(5-Carboxyindol-3-yl)-butyl]-1,2,3,4-tetrahydro-benzofuro-[2,3-c]pyridin.

Analog erhält man durch Hydrolyse der entsprechenden Nitrile:

2-[4-(4-Carboxyindol-3-yl)-butyl]-1,2,3,4-tetrahydro-benzothieno[2,3-c]pyridin
2-[4-(5-Carboxyindol-3-yl)-butyl]-1,2,3,4-tetrahydro-benzothieno[2,3-c]pyridin
2-[4-(6-Carboxyindol-3-yl)-butyl]-1,2,3,4-tetrahydro-benzothieno[2,3-c]pyridin
2-[4-(7-Carboxyindol-3-yl)-butyl]-1,2,3,4-tetrahydro-benzothieno[2,3-c]pyridin
2-[4-(4-Carboxyindol-3-yl)-butyl]-1,2,3,4-tetrahydro-benzofuro[2,3-c]pyridin
2-[4-(6-Carboxyindol-3-yl)-butyl]-1,2,3,4-tetrahydro-benzofuro[2,3-c]pyridin
2-[4-(7-Carboxyindol-3-yl)-butyl]-1,2,3,4-tetrahydro-benzofuro[2,3-c]pyridin

3-[4-(4-Carboxyindol-3-yl)-butyl]-1,2,3,4,5,6-hexahydro-benzo[f]isochinolin

3-[4-(5-Carboxyindol-3-yl)-butyl]-1,2,3,4,5,6-hexahydro-benzo[f]isochinolin

3-[4-(6-Carboxyindol-3-yl)-butyl]-1,2,3,4,5,6-hexahydro-benzo[f]isochinolin

3-[4-(7-Carboxyindol-3-yl)-butyl]-1,2,3,4,5,6-hexahydro-benzo[f]isochinolin

2-[4-(4-Carboxyindol-3-yl)-butyl]-1,2,3,4-tetrahydro-2-azafluoren

2-[4-(5-Carboxyindol-3-yl)-butyl]-1,2,3,4-tetrahydro-2-azafluoren

2-[4-(6-Carboxyindol-3-yl)-butyl]-1,2,3,4-tetrahydro-2-azafluoren

2-[4-(7-Carboxyindol-3-yl)-butyl]-1,2,3,4-tetrahydro-2-azafluoren

2-[4-(4-Carboxyindol-3-yl)-butyl]-9-hydroxy-1,2,3,4-tetrahydro-2-azafluoren

2-[4-(5-Carboxyindol-3-yl)-butyl]-9-hydroxy-1,2,3,4-tetrahydro-2-azafluoren

2-[4-(6-Carboxyindol-3-yl)-butyl]-9-hydroxy-1,2,3,4-tetrahydro-2-azafluoren

2-[4-(7-Carboxyindol-3-yl)-butyl]-9-hydroxy-1,2,3,4-tetrahydro-2-azafluoren

2-[4-(4-Carboxyindol-3-yl)-butyl]-9-oxo-1,2,3,4-tetra-hydro-2-azafluoren

2-[4-(5-Carboxyindol-3-yl)-butyl]-9-oxo-1,2,3,4-tetra-hydro-2-azafluoren

2-[4-(6-Carboxyindol-3-yl)-butyl]-9-oxo-1,2,3,4-tetra-hydro-2-azafluoren

2-[4-(7-Carboxyindol-3-yl)-butyl]-9-oxo-1,2,3,4-tetra-hydro-2-azafluoren.

Beispiel 16

Man kocht 4,96 g 3-[4-(1-Benzolsulfonylindol-3-yl)-butyl]-1,2,3,4,5,6-hexahydro-benzo[f]isochinolin [erhältlich aus 1-Benzolsulfonyl-3-(4-chlorbutyl)-indol und 1,2,3,4,5,6-Hexahydrobenzo[f]isochinolin] mit 1 g KOH in 7 ml Wasser und 14 ml Ethanol 16 Stunden, konzentriert das Gemisch, arbeitet wie üblich auf und erhält 3-[4-(Indol-3-yl)-butyl]-1,2,3,4,5,6-hexahydrobenzo[f]isochinolin, F. 190 - 192°. Hydrochlorid, F. 198 - 200°.

Beispiel 17

Man löst 2,76 g Na in 180 ml Ethanol, gibt 24,9 g 2-(2-Mercaptoethyl)-1,2,3,4-tetrahydro-benzothieno[2,3-c]pyridin [erhältlich durch Reaktion von 1,2,3,4-Tetrahydrobenzothieno[2,3-c]pyridin mit Thioglykolsäure zu 2-(2-Mercaptoacetyl)-1,2,3,4-tetrahydro-benzothieno[2,3-c]-pyridin und Reduktion mit $LiAlH_4$] und 23,2 g Gramin-5-carbonsäuremethylester hinzu, kocht 16 Stunden, dampft ein, arbeitet wie üblich auf und erhält 2-[4-(5-Methoxycarbonylindol-3-yl)-3-thiabutyl]-1,2,3,4-tetrahydrobenzothieno[2,3-c]pyridin.

Analog erhält man aus den entsprechenden Ausgangsstoffen der Formeln IV und V die folgenden 1,2,3,4-Tetrahydrobenzothieno[2,3-c]-pyridine:

2-[4-(Indol-3-yl)-3-thiabutyl]-
2-[4-(4-Cyanindol-3-yl)-3-thiabutyl]-
2-[4-(5-Cyanindol-3-yl)-3-thiabutyl]-
2-[4-(6-Cyanindol-3-yl)-3-thiabutyl]-
2-[4-(7-Cyanindol-3-yl)-3-thiabutyl]-2-[4-(4-Methoxycarbonylindol-3-yl)-3-thiabutyl]-
2-[4-(6-Methoxycarbonylindol-3-yl)-3-thiabutyl]-
2-[4-(7-Methoxycarbonylindol-3-yl)-3-thiabutyl]-
2-[4-(4-Ethoxycarbonylindol-3-yl)-3-thiabutyl]-
2-[4-(5-Ethoxycarbonylindol-3-yl)-3-thiabutyl]-
2-[4-(6-Ethoxycarbonylindol-3-yl)-3-thiabutyl]-
2-[4-(7-Ethoxycarbonylindol-3-yl)-3-thiabutyl]-
2-[4-(4-Carbamoylindol-3-yl)-3-thiabutyl]-
2-[4-(5-Carbamoylindol-3-yl)-3-thiabutyl]-
2-[4-(6-Carbamoylindol-3-yl)-3-thiabutyl]-
2-[4-(7-Carbamoylindol-3-yl)-3-thiabutyl]-
2-[4-(4-Methoxyindol-3-yl)-3-thiabutyl]-
2-[4-(5-Methoxyindol-3-yl)-3-thiabutyl]-
2-[4-(6-Methoxyindol-3-yl)-3-thiabutyl]-
2-[4-(7-Methoxyindol-3-yl)-3-thiabutyl]-
2-[4-(5-Fluorindol-3-yl)-3-thiabutyl]-
2-[4-(6-Fluorindol-3-yl)-3-thiabutyl]-
2-[4-(5-Chlorindol-3-yl)-3-thiabutyl]-
2-[4-(6-Chlorindol-3-yl)-3-thiabutyl]-
2-[4-(5-Bromindol-3-yl)-3-thiabutyl]-
2-[4-(6-Bromindol-3-yl)-3-thiabutyl]-,

die folgenden 1,2,3,4-Tetrahydrobenzofuro[2,3-c]pyridine:

2-[4-(Indol-3-yl)-3-thiabutyl]-
2-[4-(4-Cyanindol-3-yl)-3-thiabutyl]-
2-[4-(5-Cyanindol-3-yl)-3-thiabutyl]-
2-[4-(6-Cyanindol-3-yl)-3-thiabutyl]-
2-[4-(7-Cyanindol-3-yl)-3-thiabutyl]-
2-[4-(4-Methoxycarbonylindol-3-yl)-3-thiabutyl]-
2-[4-(5-Methoxycarbonylindol-3-yl)-3-thiabutyl-
2-[4-(6-Methoxycarbonylindol-3-yl)-3-thiabutyl]-
2-[4-(7-Methoxycarbonylindol-3-yl)-3-thiabutyl]-
2-[4-(4-Ethoxycarbonylindol-3-yl)-3-thiabutyl]-
2-[4-(5-Ethoxycarbonylindol-3-yl)-3-thiabutyl]-
2-[4-(6-Ethoxycarbonylindol-3-yl)-3-thiabutyl]-
2-[4-(7-Ethoxycarbonylindol-3-yl)-3-thiabutyl]-2-[4-(4-Carbamoylindol-3-yl)-3-thiabutyl]-
2-[4-(5-Carbamoylindol-3-yl)-3-thiabutyl]-
2-[4-(6-Carbamoylindol-3-yl)-3-thiabutyl]-
2-[4-(7-Carbamoylindol-3-yl)-3-thiabutyl]-
2-[4-(4-Methoxyindol-3-yl)-3-thiabutyl]-
2-[4-(5-Methoxyindol-3-yl)-3-thiabutyl]-
2-[4-(6-Methoxyindol-3-yl)-3-thiabutyl]-
2-[4-(7-Methoxyindol-3-yl)-3-thiabutyl]-
2-[4-(5-Fluorindol-3-yl)-3-thiabutyl]-
2-[4-(6-Fluorindol-3-yl)-3-thiabutyl]-
2-[4-(5-Chlorindol-3-yl)-3-thiabutyl]-
2-[4-(6-Chlorindol-3-yl)-3-thiabutyl]-
2-[4-(5-Bromindol-3-yl)-3-thiabutyl]-
2-[4-(6-Bromindol-3-yl)-3-thiabutyl]-;

die folgenden 1,2,3,4,5,6-Hexahydrobenz[f]isochinoline:

3-[4-(Indol-3-yl)-3-thiabutyl]-
3-[4-(4-Cyanindol-3-yl)-3-thiabutyl]-
3-[4-(5-Cyanindol-3-yl)-3-thiabutyl]-
3-[4-(6-Cyanindol-3-yl)-3-thiabutyl]-
3-[4-(7-Cyanindol-3-yl)-3-thiabutyl]-
3-[4-(4-Methoxycarbonylindol-3-yl)-3-thiabutyl]-
3-[4-(5-Methoxycarbonylindol-3-yl)-3-thiabutyl]-
3-[4-(6-Methoxycarbonylindol-3-yl)-3-thiabutyl]-
3-[4-(7-Methoxycarbonylindol-3-yl)-3-thiabutyl]-
3-[4-(4-Ethoxycarbonylindol-3-yl)-3-thiabutyl]-
3-[4-(5-Ethoxycarbonylindol-3-yl)-3-thiabutyl]-
3-[4-(6-Ethoxycarbonylindol-3-yl)-3-thiabutyl]-
3-[4-(7-Ethoxycarbonylindol-3-yl)-3-thiabutyl]-
3-[4-(4-Carbamoylindol-3-yl)-3-thiabutyl]-
3-[4-(5-Carbamoylindol-3-yl)-3-thiabutyl]-
3-[4-(6-Carbamoylindol-3-yl)-3-thiabutyl]-
3-[4-(7-Carbamoylindol-3-yl)-3-thiabutyl]-
3-[4-(4-Methoxyindol-3-yl)-3-thiabutyl]-3-[4-(5-Methoxyindol-3-yl)-3-thiabutyl]-
3-[4-(6-Methoxyindol-3-yl)-3-thiabutyl]-
3-[4-(7-Methoxyindol-3-yl)-3-thiabutyl]-
3-[4-(5-Fluorindol-3-yl)-3-thiabutyl]-
3-[4-(6-Fluorindol-3-yl)-3-thiabutyl]-
3-[4-(5-Chlorindol-3-yl)-3-thiabutyl]-
3-[4-(6-Chlorindol-3-yl)-3-thiabutyl]-
3-[4-(5-Bromindol-3-yl)-3-thiabutyl]-
3-[4-(6-Bromindol-3-yl)-3-thiabutyl]-;

die folgenden 1,2,3,4-Tetrahydro-2-azafluorene:

2-[4-(Indol-3-yl)-3-thiabutyl]-
2-[4-(4-Cyanindol-3-yl)-3-thiabutyl]-
2-[4-(5-Cyanindol-3-yl)-3-thiabutyl]-
2-[4-(6-Cyanindol-3-yl)-3-thiabutyl]-
2-[4-(7-Cyanindol-3-yl)-3-thiabutyl]-
2-[4-(4-Methoxycarbonylindol-3-yl)-3-thiabutyl]-
2-[4-(5-Methoxycarbonylindol-3-yl)-3-thiabutyl-
2-[4-(6-Methoxycarbonylindol-3-yl)-3-thiabutyl]-
2-[4-(7-Methoxycarbonylindol-3-yl)-3-thiabutyl]-
2-[4-(4-Ethoxycarbonylindol-3-yl)-3-thiabutyl]-
2-[4-(5-Ethoxycarbonylindol-3-yl)-3-thiabutyl]-
2-[4-(6-Ethoxycarbonylindol-3-yl)-3-thiabutyl]-
2-[4-(7-Ethoxycarbonylindol-3-yl)-3-thiabutyl]-
2-[4-(4-Carbamoylindol-3-yl)-3-thiabutyl]-
2-[4-(5-Carbamoylindol-3-yl)-3-thiabutyl]-
2-[4-(6-Carbamoylindol-3-yl)-3-thiabutyl]-
2-[4-(7-Carbamoylindol-3-yl)-3-thiabutyl]-
2-[4-(4-Methoxyindol-3-yl)-3-thiabutyl]-
2-[4-(5-Methoxyindol-3-yl)-3-thiabutyl]-
2-[4-(6-Methoxyindol-3-yl)-3-thiabutyl]-
2-[4-(7-Methoxyindol-3-yl)-3-thiabutyl]-2-[4-(5-Fluorindol-3-yl)-3-thiabutyl]-
2-[4-(6-Fluorindol-3-yl)-3-thiabutyl]-
2-[4-(5-Chlorindol-3-yl)-3-thiabutyl]-
2-[4-(6-Chlorindol-3-yl)-3-thiabutyl]-
2-[4-(5-Bromindol-3-yl)-3-thiabutyl]-
2-[4-(6-Bromindol-3-yl)-3-thiabutyl]-;

die folgenden 9-Hydroxy-1,2,3,4-tetrahydro-2-azafluorene:

2-[4-(Indol-3-yl)-3-thiabutyl]-
2-[4-(4-Cyanindol-3-yl)-3-thiabutyl]-
2-[4-(5-Cyanindol-3-yl)-3-thiabutyl]-
2-[4-(6-Cyanindol-3-yl)-3-thiabutyl]-
2-[4-(7-Cyanindol-3-yl)-3-thiabutyl]-
2-[4-(4-Methoxycarbonylindol-3-yl)-3-thiabutyl]-
2-[4-(5-Methoxycarbonylindol-3-yl)-3-thiabutyl]-
2-[4-(6-Methoxycarbonylindol-3-yl)-3-thiabutyl]-
2-[4-(7-Methoxycarbonylindol-3-yl)-3-thiabutyl]-
2-[4-(4-Ethoxycarbonylindol-3-yl)-3-thiabutyl]-
2-[4-(5-Ethoxycarbonylindol-3-yl)-3-thiabutyl]-
2-[4-(6-Ethoxycarbonylindol-3-yl)-3-thiabutyl]-
2-[4-(7-Ethoxycarbonylindol-3-yl)-3-thiabutyl]-
2-[4-(4-Carbamoylindol-3-yl)-3-thiabutyl]-
2-[4-(5-Carbamoylindol-3-yl)-3-thiabutyl]-
2-[4-(6-Carbamoylindol-3-yl)-3-thiabutyl]-
2-[4-(7-Carbamoylindol-3-yl)-3-thiabutyl]-
2-[4-(4-Methoxyindol-3-yl)-3-thiabutyl]-
2-[4-(5-Methoxyindol-3-yl)-3-thiabutyl]-
2-[4-(6-Methoxyindol-3-yl)-3-thiabutyl]-
2-[4-(7-Methoxyindol-3-yl)-3-thiabutyl]-
2-[4-(5-Fluorindol-3-yl)-3-thiabutyl]-
2-[4-(6-Fluorindol-3-yl)-3-thiabutyl]-
2-[4-(5-Chlorindol-3-yl)-3-thiabutyl]-
2-[4-(6-Chlorindol-3-yl)-3-thiabutyl]-
2-[4-(5-Bromindol-3-yl)-3-thiabutyl]-
2-[4-(6-Bromindol-3-yl)-3-thiabutyl]-;

die folgenden 9-Oxo-1,2,3,4-tetrahydro-2-azafluorene:

2-[4-(Indol-3-yl)-3-thiabutyl]-
2-[4-(4-Cyanindol-3-yl)-3-thiabutyl]-
2-[4-(5-Cyanindol-3-yl)-3-thiabutyl]-
2-[4-(6-Cyanindol-3-yl)-3-thiabutyl]-
2-[4-(7-Cyanindol-3-yl)-3-thiabutyl]-
2-[4-(4-Methoxycarbonylindol-3-yl)-3-thiabutyl]-
2-[4-(5-Methoxycarbonylindol-3-yl)-3-thiabutyl]-
2-[4-(6-Methoxycarbonylindol-3-yl)-3-thiabutyl]-
2-[4-(7-Methoxycarbonylindol-3-yl)-3-thiabutyl]-
2-[4-(4-Ethoxycarbonylindol-3-yl)-3-thiabutyl]-
2-[4-(5-Ethoxycarbonylindol-3-yl)-3-thiabutyl]-
2-[4-(6-Ethoxycarbonylindol-3-yl)-3-thiabutyl]-
2-[4-(7-Ethoxycarbonylindol-3-yl)-3-thiabutyl]-
2-[4-(4-Carbamoylindol-3-yl)-3-thiabutyl]-
2-[4-(5-Carbamoylindol-3-yl)-3-thiabutyl]-
2-[4-(6-Carbamoylindol-3-yl)-3-thiabutyl]-
2-[4-(7-Carbamoylindol-3-yl)-3-thiabutyl]-
2-[4-(4-Methoxyindol-3-yl)-3-thiabutyl]-
2-[4-(5-Methoxyindol-3-yl)-3-thiabutyl]-
2-[4-(6-Methoxyindol-3-yl)-3-thiabutyl]-
2-[4-(7-Methoxyindol-3-yl)-3-thiabutyl]-
2-[4-(5-Fluorindol-3-yl)-3-thiabutyl]-
2-[4-(6-Fluorindol-3-yl)-3-thiabutyl]-
2-[4-(5-Chlorindol-3-yl)-3-thiabutyl]-
2-[4-(6-Chlorindol-3-yl)-3-thiabutyl]-
2-[4-(5-Bromindol-3-yl)-3-thiabutyl]-
2-[4-(6-Bromindol-3-yl)-3-thiabutyl]-.

Beispiel 18

Eine Lösung von 5,09 g 2-[4-(5-Dimethyl-tert.-butyl-silyloxy-indol-3-yl)-3-thiabutyl]-1,2,3,4-tetrahydro-benzothieno[2,3-c]pyridin [erhältlich durch Veretherung

von 5-Hydroxyindol zu 5-Dimethyl-tert.-butylsilyloxy-indol (Rf 0,46, Dichlormethan), Mannich-Reaktion zu 5-Dimethyl-tert.-butylsilyloxy-gramin (Hydrochlorid, F. 156 - 157°) und Umsetzung mit 2-(2-Mercaptoethyl)-1,2,3,4-tetrahydro-benzothieno[2,3-c]pyridin] und 1,16 g KF in 200 ml Methanol wird 16 Std. bei 20° stehengelassen. Nach üblicher Aufarbeitung erhält man 2-[4-(5-Hydroxyindol-3-yl)-3-thiabutyl]-1,2,3,4-tetrahydro-benzothieno[2,3-c]pyridin.

Analog erhält man aus 4-, 6- oder 7-Hydroxyindol über 4- (Rf 0,52, Dichlormethan), 6- (Rf 0,50, Dichlormethan) oder 7-Dimethyl-tert.-butylsilyloxy-indol, 4- (Hydrochlorid, F. 168 - 170°), 6- (Hydrochlorid, F. 161 - 162°) oder 7-Dimethyl-tert.-butylsilyloxy-gramin und 2-[4-(4-, 2-[4-(6- oder 2-[4-(7-Dimethyl-tert.-butylsilyloxy-indol-3-yl)-3-thiabutyl]-1,2,3,4-tetrahydro-benzothieno[2,3-c]-pyridin die folgenden 1,2,3,4-Tetrahydro-benzothieno-[2,3-c]pyridine:

2-[4-(4-Hydroxyindol-3-yl)-3-thiabutyl]-
2-[4-(6-Hydroxyindol-3-yl)-3-thiabutyl]-
2-[4-(7-Hydroxyindol-3-yl)-3-thiabutyl]-.

Analog erhält man mit den entsprechenden Ausgangsstoffen der Formel IIIa die nachstehenden 1,2,3,4-Tetrahydro-benzofuro[2,3-c]pyridine:

2-[4-(4-Hydroxyindol-3-yl)-3-thiabutyl]-
2-[4-(5-Hydroxyindol-3-yl)-3-thiabutyl]-
2-[4-(6-Hydroxyindol-3-yl)-3-thiabutyl]-
2-[4-(7-Hydroxyindol-3-yl)-3-thiabutyl]-;

die nachstehenden 1,2,3,4,5,6-Hexahydrobenz[f]isochinoline:

2-[4-(4-Hydroxyindol-3-yl)-3-thiabutyl]-
2-[4-(5-Hydroxyindol-3-yl)-3-thiabutyl]-
2-[4-(6-Hydroxyindol-3-yl)-3-thiabutyl]-
2-[4-(7-Hydroxyindol-3-yl)-3-thiabutyl]-;

die nachstehenden 1,2,3,4-Tetrahydro-2-azafluorene:

2-[4-(4-Hydroxyindol-3-yl)-3-thiabutyl]-
2-[4-(5-Hydroxyindol-3-yl)-3-thiabutyl]-
2-[4-(6-Hydroxyindol-3-yl)-3-thiabutyl]-
2-[4-(7-Hydroxyindol-3-yl)-3-thiabutyl]-
2-[4-(4-Hydroxyindol-3-yl)-3-thiabutyl]-9-hydroxy-
2-[4-(5-Hydroxyindol-3-yl)-3-thiabutyl]-9-hydroxy-
2-[4-(6-Hydroxyindol-3-yl)-3-thiabutyl]-9-hydroxy-
2-[4-(7-Hydroxyindol-3-yl)-3-thiabutyl]-9-hydroxy-
2-[4-(4-Hydroxyindol-3-yl)-3-thiabutyl]-9-oxo-
2-[4-(5-Hydroxyindol-3-yl)-3-thiabutyl]-9-oxo-
2-[4-(6-Hydroxyindol-3-yl)-3-thiabutyl]-9-oxo-
2-[4-(7-Hydroxyindol-3-yl)-3-thiabutyl]-9-oxo-.

Beispiel 19

Ein Gemisch von 3,9 g 2-[4-(5-Methoxyindol-3-yl)-butyl]-1,2,3,4-tetrahydro-benzothieno[2,3-c]pyridin und 3,5 g Pyridinhydrochlorid wird 3 Stunden bei 160° gerührt. Nach üblicher Aufarbeitung erhält man 2-[4-(5-Hydroxyindol-3-yl)-butyl]-1,2,3,4-tetrahydro-benzothieno[2,3-c]-pyridin.

Beispiel 20

Man löst 37,4 g 2-[4-(5-Hydroxymethylindol-3-yl)-butyl]-1,2,3,4-tetrahydro-benzofuro[2,3-c]pyridin in 1,6 l THF und gibt 300 ml Ether hinzu. Unter Rühren werden 55 g $MnO_2$ zugegeben. Man rührt 16 Std. bei 20°, gibt portionsweise weitere 100 g $MnO_2$ hinzu und rührt weitere 100 Std. bei 20°. Nach Filtration und üblichem Aufarbeiten erhält man 2-[4-(5-Formylindol-3-yl)-butyl]-1,2,3,4-tetrahydro-benzofuro[2,3-c]pyridin.

Beispiel 21

Man löst 3,74 g 2-[4-(5-Hydroxymethylindol-3-yl)-butyl]-1,2,3,4-tetrahydro-benzofuro[2,3-c]pyridin in 50 ml Dichlormethan, versetzt die Lösung mit 9 g $MnO_2$, rührt 60 Std. bei 40° und filtriert die unlöslichen Anteile ab. Nach üblicher Aufarbeitung des Filtrats erhält man 2-[4-(5-Carboxyindol-3-yl)-butyl]-1,2,3,4-tetrahydro-benzofuro[2,3-c]pyridin.

Beispiel 22

Man löst 3,72 g 2-[4-(5-Formylindol-3-yl)-butyl]-1,2,3,4-tetrahydro-benzofuro[2,3-c]pyridin in 80 ml Dichlormethan, versetzt mit 9 g $MnO_2$ und rührt die Suspension 48 Std. bei 40°. Nach Filtrieren und üblicher Aufarbeitung erhält man 2-[4-(5-Carboxyindol-3-yl)-butyl]-1,2,3,4-tetrahydro-benzofuro[2,3-c]pyridin.

Beispiel 23

Man suspendiert 3,88 g 2-[4-(5-Carboxyindol-3-yl)-butyl]-1,2,3,4-tetrahydro-benzofuro[2,3-c]pyridin in 25 ml Toluol und tropft unter $N_2$ und Rühren die 3fach molare Menge einer 20 %igen Lösung von Diisobutylaluminiumhydrid in Toluol zu, kocht 2 Std., kühlt ab, zersetzt das Gemisch mit Wasser, arbeitet wie üblich auf und erhält 2-[4-(5-Formylindol-3-yl)-butyl]-1,2,3,4-tetrahydro-benzofuro[2,3-c]pyridin.

Beispiel 24

Zu einer Suspension von 0,76 g Lithiumaluminiumhydrid in 30 ml THF wird unter Rühren und $N_2$ eine Lösung von 3,88 g 2-[4-(5-Carboxyindol-3-yl)-butyl]-1,2,3,4-tetrahydro-benzofuro[2,3-c]pyridin in 40 ml THF zugetropft. Man rührt noch 2 Std. bei 20°, zersetzt mit verdünnter Natronlauge, dann mit Wasser und filtriert. Das Filtrat wird wie üblich aufgearbeitet. Man erhält 2-[4-(5-Hydroxymethylindol-3-yl)-butyl]-1,2,3,4-tetrahydro-benzofuro-[2,3-c]pyridin.

Beispiel 25

Zu einer Suspension von 0,57 g Lithiumaluminiumhydrid in 20 ml THF wird unter Rühren und $N_2$ bei 20° eine Lösung von 4,18 g 2-[4-(5-Methoxycarbonylindol-3-yl)-butyl]-1,2,3,4-tetrahydro-benzothieno[2,3-c]pyridin in 40 ml THF zugetropft. Man rührt 1 Std. bei 20°, zersetzt mit verdünnter Natronlauge, dann mit Wasser, filtriert, arbeitet das Filtrat wie üblich auf und erhält 2-[4-(5-Hydroxymethylindol-3-yl)-butyl]-1,2,3,4-tetrahydro-benzothieno[2,3-c]pyridin.

Analog erhält man durch Reduktion der entsprechenden Ester:

2-[4-(4-Hydroxymethylindol-3-yl)-butyl]-1,2,3,4-tetrahydro-benzothieno[2,3-c]pyridin

2-[4-(6-Hydroxymethylindol-3-yl)-butyl]-1,2,3,4-tetrahydro-benzothieno[2,3-c]pyridin

2-[4-(7-Hydroxymethylindol-3-yl)-butyl]-1,2,3,4-tetrahydro-benzothieno[2,3-c]pyridin

2-[4-(4-Hydroxymethylindol-3-yl)-butyl]-1,2,3,4-tetrahydro-benzofuro[2,3-c]pyridin

2-[4-(5-Hydroxymethylindol-3-yl)-butyl]-1,2,3,4-tetrahydro-benzofuro[2,3-c]pyridin

2-[4-(6-Hydroxymethylindol-3-yl)-butyl]-1,2,3,4-tetrahydro-benzofuro[2,3-c]pyridin

2-[4-(7-Hydroxymethylindol-3-yl)-butyl]-1,2,3,4-tetrahydro-benzofuro[2,3-c]pyridin

3-[4-(4-Hydroxymethylindol-3-yl)-butyl]-1,2,3,4,5,6-hexahydro-benz[f]isochinolin

3-[4-(5-Hydroxymethylindol-3-yl)-butyl]-1,2,3,4,5,6-hexahydro-benz[f]isochinolin

3-[4-(6-Hydroxymethylindol-3-yl)-butyl]-1,2,3,4,5,6-hexahydro-benz[f]isochinolin

3-[4-(7-Hydroxymethylindol-3-yl)-butyl]-1,2,3,4,5,6-hexahydro-benz[f]isochinolin

2-[4-(4-Hydroxymethylindol-3-yl)-butyl]-1,2,3,4-tetrahydro-2-azafluoren

2-[4-(5-Hydroxymethylindol-3-yl)-butyl]-1,2,3,4-tetrahydro-2-azafluoren

2-[4-(6-Hydroxymethylindol-3-yl)-butyl]-1,2,3,4-tetrahydro-2-azafluoren

2-[4-(7-Hydroxymethylindol-3-yl)-butyl]-1,2,3,4-tetrahydro-2-azafluoren

2-[4-(4-Hydroxymethylindol-3-yl)-butyl]-9-hydroxy-1,2,3,4-tetrahydro-2-azafluoren

2-[4-(5-Hydroxymethylindol-3-yl)-butyl]-9-hydroxy-1,2,3,4-tetrahydro-2-azafluoren

2-[4-(6-Hydroxymethylindol-3-yl)-butyl]-9-hydroxy-1,2,3,4-tetrahydro-2-azafluoren

2-[4-(7-Hydroxymethylindol-3-yl)-butyl]-9-hydroxy-1,2,3,4-tetrahydro-2-azafluoren

2-[4-(4-Hydroxymethylindol-3-yl)-3-thiabutyl]-1,2,3,4-tetrahydro-benzothieno[2,3-c]pyridin

2-[4-(5-Hydroxymethylindol-3-yl)-3-thiabutyl]-1,2,3,4-tetrahydro-benzothieno[2,3-c]pyridin

2-[4-(6-Hydroxymethylindol-3-yl)-3-thiabutyl]-1,2,3,4-tetrahydro-benzothieno[2,3-c]pyridin

2-[4-(7-Hydroxymethylindol-3-yl)-3-thiabutyl]-1,2,3,4-tetrahydro-benzothieno[2,3-c]pyridin

2-[4-(4-Hydroxymethylindol-3-yl)-3-thiabutyl]-1,2,3,4-tetrahydro-benzofuro[2,3-c]pyridin

2-[4-(5-Hydroxymethylindol-3-yl)-3-thiabutyl]-1,2,3,4-tetrahydro-benzofuro[2,3-c]pyridin

2-[4-(6-Hydroxymethylindol-3-yl)-3-thiabutyl]-1,2,3,4-tetrahydro-benzofuro[2,3-c]pyridin

2-[4-(7-Hydroxymethylindol-3-yl)-3-thiabutyl]-1,2,3,4-tetrahydro-benzofuro[2,3-c]pyridin

3-[4-(4-Hydroxymethylindol-3-yl)-3-thiabutyl]-1,2,3,4,5,6-hexahydrobenz[f]isochinolin

3-[4-(5-Hydroxymethylindol-3-yl)-3-thiabutyl]-1,2,3,4,5,6-hexahydrobenz[f]isochinolin

3-[4-(6-Hydroxymethylindol-3-yl)-3-thiabutyl]-1,2,3,4,5,6-hexahydrobenz[f]isochinolin

3-[4-(7-Hydroxymethylindol-3-yl)-3-thiabutyl]-1,2,3,4,5,6-hexahydrobenz[f]isochinolin

2-[4-(4-Hydroxymethylindol-3-yl)-3-thiabutyl]-1,2,3,4-tetrahydro-2-azafluoren

2-[4-(5-Hydroxymethylindol-3-yl)-3-thiabutyl]-1,2,3,4-tetrahydro-2-azafluoren

2-[4-(6-Hydroxymethylindol-3-yl)-3-thiabutyl]-1,2,3,4-tetrahydro-2-azafluoren

2-[4-(7-Hydroxymethylindol-3-yl)-3-thiabutyl]-1,2,3,4-tetrahydro-2-azafluoren

2-[4-(4-Hydroxymethylindol-3-yl)-3-thiabutyl]-9-hydroxy-1,2,3,4-tetrahydro-2-azafluoren

2-[4-(5-Hydroxymethylindol-3-yl)-3-thiabutyl]-9-hydroxy-1,2,3,4-tetrahydro-2-azafluoren

2-[4-(6-Hydroxymethylindol-3-yl)-3-thiabutyl]-9-hydroxy-1,2,3,4-tetrahydro-2-azafluoren

2-[4-(7-Hydroxymethylindol-3-yl)-3-thiabutyl]-9-hydroxy-1,2,3,4-tetrahydro-2-azafluoren.

Beispiel 26

Zu einer Suspension von 0,57 g Lithiumaluminiumhydrid in 20 ml THF wird unter $N_2$ und Rühren eine Lösung von 3,84 g 3-[4-(6-Formylindol-3-yl)-butyl]-1,2,3,4,5,6-hexahydro-benz[f]isochinolin in 40 ml THF zugetropft. Man rührt noch 1 Std. bei 20°, zersetzt mit verdünnter Natronlauge, dann mit Wasser, filtriert ab, arbeitet wie üblich auf und erhält 3-[4-(6-Hydroxymethylindol-3-yl)-butyl]-1,2,3,4,5,6-hexahydro-benz[f]isochinolin.

Beispiel 27

In eine Lösung von 3,88 g 2-[4-(5-Carboxyindol-3-yl)-butyl]-1,2,3,4-tetrahydro-benzofuro[2,3-c]pyridin in 50 ml absolutem Ethanol wird 2 Std. HCl eingeleitet. Man läßt noch 48 Std. bei 20° stehen, arbeitet wie üblich auf und erhält 2-[4-(5-Ethoxycarbonylindol-3-yl)-butyl]-1,2,3,4-tetrahydro-benzofuro[2,3-c]pyridin.

Beispiel 28

Eine Lösung von 4 g 3-[4-(5-Carboxyindol-3-yl)-butyl]-1,2,3,4,5,6-hexahydrobenz[f]isochinolin in 40 ml DMF wird mit 1,8 g N,N'-Carbonyldiimidazol versetzt, 2 Std. bei 20° gerührt, dann mit 0,6 g $NH_4Cl$ versetzt und 3 Std. auf 40° erwärmt. Nach üblicher Aufarbeitung erhält man 3-[4-(5-Carbamoylindol-3-yl)-butyl]-1,2,3,4,5,6-hexahydrobenz[f]isochinolin.

Beispiel 29

Man löst 3,88 g 2-[4-(5-Carboxyindol-3-yl)-butyl]-1,2,3,4-tetrahydro-benzofuro[2,3-c]pyridin in 30 ml Chloroform, sättigt mit HCl-Gas, tropft 1,8 g Thionylchlorid hinzu und kocht 2 Std. Man dampft ein, gibt 30 ml Toluol hinzu, dampft erneut ein, löst das erhaltene rohe Säurechlorid in 20 ml Chloroform, tropft diese Lösung unter Rühren zu einer gesättigten Lösung von Ammoniak in 50 ml Chloroform, rührt 2 Std. bei 20°, filtriert und konzentriert das Filtrat. Nach üblicher Aufarbeitung erhält man 2-[4-(5-Carbamoylindol-3-yl)-butyl]-1,2,3,4-tetrahydrobenzofuro[2,3-c]pyridin.

Beispiel 30

Zu einer Lösung von 4,02 g 2-[4-(5-Methoxycarbonylindol-3-yl)-butyl]-1,2,3,4-tetrahydro-benzofuro[2,3-c]pyridin in 30 ml Dimethylformamid werden 0,02 mol konz. Ammoniak (D = 0,9) bei 20° zugetropft. Man rührt 1 Std. bei 20° nach, arbeitet wie üblich auf und erhält 2-[4-(5-Carbamoylindol-3-yl)-butyl]-1,2,3,4-tetrahydro-benzofuro-[2,3-c]pyridin.

Beispiel 31

Ein Gemisch von 38,7 g 2-[4-(5-Carbamoylindol-3-yl)-butyl]-1,2,3,4-tetrahydro-benzofuro[2,3-c]pyridin, 27,1 g NaOH, 525 ml Wasser und 450 ml Diethylenglykolmonoethylether wird 16 Std. unter Rühren gekocht. Man kühlt ab, arbeitet wie üblich auf, säuert an und erhält 2-[4-(5-Carboxyindol-3-yl)-butyl]-1,2,3,4-tetrahydro-benzofuro-[2,3-c]pyridin.

Beispiel 32

Man kocht 4,16 g 2-[4-(5-Ethoxycarbonylindol-3-yl)-butyl]-1,2,3,4-tetrahydrobenzofuro[2,3-c]pyridin 30 Min. mit 20 ml Wasser und 100 ml 2n ethanolischer KOH, arbeitet wie üblich auf und erhält 2-[4-(5-Carboxyindol-3-yl)-butyl]-1,2,3,4-tetrahydro-benzofuro[2,3-c]pyridin.

Beispiel 33

Analog Beispiel 1 erhält man mit 1,2,3,4-Tetrahydrobenz-[f]isochinolin das 3-[4-(Indol-3-yl)-butyl]-1,2,3,4-tetrahydro-benz[f]isochinolin.

Analog erhält man die folgenden 1,2,3,4-Tetrahydro-benz[f]-isochinoline:

3-[2-(Indol-3-yl)-ethyl]-
3-[3-(Indol-3-yl)-propyl]-
3-[4-(5,6-Methylendioxyindol-3-yl)-butyl]-
3-[4-(4-Cyanindol-3-yl)-butyl]-
3-[4-(5-Cyanindol-3-yl)-butyl]-
3-[4-(6-Cyanindol-3-yl)-butyl]-
3-[4-(7-Cyanindol-3-yl)-butyl]-3-[4-(4-Methoxycarbonylindol-3-yl)-butyl]-
3-[4-(5-Methoxycarbonylindol-3-yl)-butyl]-
3-[4-(6-Methoxycarbonylindol-3-yl)-butyl]-
3-[4-(7-Methoxycarbonylindol-3-yl)-butyl]-
3-[4-(4-Ethoxycarbonylindol-3-yl)-butyl]-
3-[4-(5-Ethoxycarbonylindol-3-yl)-butyl]-
3-[4-(6-Ethoxycarbonylindol-3-yl)-butyl]-
3-[4-(7-Ethoxycarbonylindol-3-yl)-butyl]-
3-[4-(4-Carbamoylindol-3-yl)-butyl]-
3-[4-(5-Carbamoylindol-3-yl)-butyl]-
3-[4-(6-Carbamoylindol-3-yl)-butyl]-
3-[4-(7-Carbamoylindol-3-yl)-butyl]-
3-[4-(5-N-Methylcarbamoylindol-3-yl)-butyl]-
3-[4-(5-N,N-Dimethylcarbamoylindol-3-yl)-butyl]-
3-[4-(1-Methylindol-3-yl)-butyl]-
3-[4-(2-Methylindol-3-yl)-butyl]-
3-[4-(5-Methylindol-3-yl)-butyl]-
3-[4-(4-Methoxyindol-3-yl)-butyl]-
3-[4-(5-Methoxyindol-3-yl)-butyl]-, F. 111-113 °
3-[4-(6-Methoxyindol-3-yl)-butyl]-
3-[4-(7-Methoxyindol-3-yl)-butyl]-
3-[4-(5,6-Dimethoxyindol-3-yl)-butyl]-
3-[4-(4-Hydroxyindol-3-yl)-butyl]-
3-[4-(5-Hydroxyindol-3-yl)-butyl]-, F. 185-186 °
3-[4-(6-Hydroxyindol-3-yl)-butyl]-
3-[4-(7-Hydroxyindol-3-yl)-butyl]-
3-[4-(5,6-Dihydroxyindol-3-yl)-butyl]-
3-[4-(5-Fluorindol-3-yl)-butyl]-
3-[4-(6-Fluorindol-3-yl)-butyl]-
3-[4-(5-Chlorindol-3-yl)-butyl]-
3-[4-(6-Chlorindol-3-yl)-butyl]-
3-[4-(5-Bromindol-3-yl)-butyl]-
3-[4-(6-Bromindol-3-yl)-butyl]-
3-[5-(Indol-3-yl)-pentyl]-.

Die nachstehenden Beispiele betreffen pharmazeutische Zubereitungen, die Verbindungen der Formel I oder ihre physiologisch unbedenklichen Salze enthalten:

Beispiel A: Tabletten

Ein Gemisch von 1 kg 2-[4-(5-Hydroxyindol-3-yl)-butyl]-1,2,3,4-tetrahydro-benzothieno[2,3-c]pyridin, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

2 kg 2-[4(5-Hydroxyindol-3-yl)-butyl]-1,2,3,4-tetrahydro-benzofuro[2,3-c]pyridin werden in üblicher Weise in Hartgelatinekapseln gefüllt, so daß jede Kapsel 20 mg des Wirkstoffs enthält.

Beispiel D: Ampullen

Eine Lösung von 1 kg 3-[4-(5-Hydroxyindol-3-yl)-butyl]-1,2,3,4,5,6-hexahydro-benz[f]isochinolin-hydrochlorid in 60 l zweifach destilliertem Wasser wird steril filtriert, in Ampullen abgefüllt, unter sterilen Bedingungen

lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihrer physiologisch unbedenklichen Säureadditionssalze enthalten.

Merck Patent Gesellschaft
mit beschränkter Haftung

6100 Darmstadt

## Patentansprüche

1. Indolderivate der allgemeinen Formel I

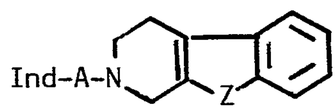

I

worin

Ind einen Indol-3-ylrest, der durch eine Hydroxymethyl-, Methylendioxy-, S-Alkyl-, SO-Alkyl-, $SO_2$-Alkyl-, CN- oder COW- Gruppe und/oder ein- oder zweifach durch Alkyl, O-Alkyl, OH, F, Cl oder Br substituiert sein kann,

W H, OH, OAlkyl, $NH_2$, NHAlkyl oder $N(Alkyl)_2$,

A $-(CH_2)_n-$, $-CH_2-S-CH_2CH_2-$, $-CH_2-SO-CH_2CH_2-$ oder $-CH_2-SO_2-CH_2CH_2-$,

Z $-(CH_2)_m-$, -CH=CH-, -CHOH-, -CO-, S, SO, $SO_2$, oder O,

n 2, 3, 4 oder 5 und

m 1, 2 oder 3

bedeuten,

0206225



worin die Alkylgruppen jeweils 1 - 4 C-Atome besitzen,
sowie deren Salze.

2. 2-[4-(5-Hydroxyindol-3-yl)-butyl]-1,2,3,4-tetra-hydro-benzothieno[2,3-c]pyridin.

3. 2-[4-(5-Hydroxyindol-3-yl)-butyl]-1,2,3,4-tetra-hydro-benzofuro[2,3-c]pyridin.

4. 3-[4-(5-Hydroxyindol-3-yl)-butyl]-1,2,3,4,5,6-hexahydrobenz[f]isochinolin.

5. Verfahren zur Herstellung von Indolderivaten der Formel I nach Anspruch 1 sowie von deren Salzen, dadurch gekennzeichnet, daß man eine Verbindung der Formel II

$$\text{Ind-A-X}^1 \qquad \text{II}$$

worin
$X^1$ X oder $NH_2$ und
X Cl, Br, J, OH oder eine reaktionsfähig funktionell abgewandelte OH-Gruppe bedeuten und
Ind und A die angegebenen Bedeutungen haben,
mit einer Verbindung der Formel III

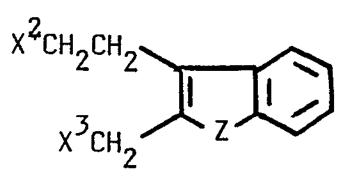

III

worin

$X^2$ und $X^3$ gleich oder verschieden sein können und, falls $X^1 = NH_2$ ist, jeweils X, andernfalls zusammen NH bedeuten und

Z die angegebene Bedeutung hat,

umsetzt

oder daß man eine sonst der Formel I entsprechende Verbindung, die jedoch an Stelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt

oder daß man eine sonst der Formel I entsprechende Verbindung, die jedoch an Stelle eines oder mehrerer Wasserstoffatome oder an Stelle einer OH-Gruppe eine oder mehrere solvolysierbare Gruppe(n) enthält, mit einem solvolysierenden Mittel behandelt

oder daß man zur Herstellung von Thioethern der Formel I, worin A $-CH_2-S-CH_2CH_2-$ bedeutet, eine Verbindung der Formel IV

$$Ind-CH_2N(R)_2 \qquad IV$$

worin

R Alkyl mit 1- 4 C-Atomen, beide Reste R zusammen auch $-(CH_2)_p-$ oder $-CH_2CH_2OCH_2CH_2-$ und

p 4 oder 5

bedeuten und

Ind die angegebene Bedeutung hat
mit einem Thiol der allgemeinen Formel V

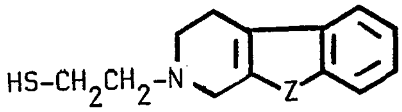

V

worin
Z die angegebene Bedeutung hat
oder einem seiner Salze
umsetzt

und/oder daß man gegebenenfalls in einer Verbindung der Formel I eine Thioethergruppe zu einer SO-Gruppe oder $SO_2$-Gruppe oder eine SO-Gruppe zu einer $SO_2$-Gruppe oxydiert und/oder eine Alkoxygruppe unter Bildung einer OH-Gruppe spaltet und/oder eine COW-Gruppe oder CN-Gruppe durch Oxydation, Reduktion, Veresterung, Amidierung oder Solvolyse in eine (andere) COW-Gruppe umwandelt und/oder eine COW-Gruppe bzw. CO-Gruppe zu einer Hydroxymethyl- bzw. zu einer CHOH- oder $CH_2$-Gruppe reduziert und/oder eine Hydroxymethyl- bzw. CHOH- Gruppe zu einer CHO- oder COOH-Gruppe bzw. zu einer CO-Gruppe oxydiert und/oder daß man eine erhaltene Base der Formel I durch Behandeln mit einer Säure in eines ihrer Säureadditionssalze oder eine Säure der Formel I durch Behandeln mit einer Base in eines ihre Metall- oder Ammoniumsalze umwandelt.

6. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger-oder Hilfsstoff und gegebenenfalls in Kombination mit einem oder mehreren weiteren Wirkstoffen in eine geeignete Dosierungsform bringt.

7. Pharmazeutische Zubereitung, gekennzeichnet durch einen Gehalt an mindestens einer Verbindung der Formel I und/oder einem ihrer physiologisch unbedenklichen Salze.

8. Verbindungen der Formel I oder ihre physiologisch unbedenklichen Salze zur Bekämpfung von Krankheiten.

9. Verwendung von Verbindungen der Formel I oder ihren physiologisch unbedenklichen Salzen bei der Bekämpfung von Krankheiten.

10. Verwendung von Verbindungen der Formel I oder ihren physiologisch unbedenklichen Salzen zur Herstellung von Arzneimitteln.

11. Indolderivate der allgemeinen Formel

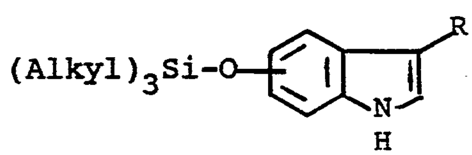

worin

R, H oder $-CH_2N(Alkyl)_2$ bedeutet und die Alkylgruppen gleich oder voneinander verschieden sein können und jeweils 1-4 C-Atome enthalten.